# EUROPEAN PATENT APPLICATION

(11) **EP 4 286 391 A1**
(43) Date of publication of application: **06.12.2023**
(21) Application number: 22745222.4
(22) Date of filing: 25.01.2022
(51) Int. Cl.: C07D 498/04, C07D 498/14, A61K 31/553, A61K 31/554, A61K 31/4162, A61P 35/00

(54) **TRICYCLIC COMPOUNDS AND USE THEREOF**

(30) Priority: 29.01.2021 CN 202110134377; 07.01.2022 CN 202210020761
(71) Applicant: Medshine Discovery Inc., Nanjing, Jiangsu 210032 (CN)
(72) Inventor: HUANG, Jingjie, Shanghai 200131 (CN); YU, Tao, Shanghai 200131 (CN); TAN, Ye, Shanghai 200131 (CN); WU, Chengde, Shanghai 200131 (CN); CHEN, Shuhui, Shanghai 200131 (CN)
(74) Representative: Calysta NV
(86) International application number: PCT/CN2022/073734
(87) International publication number: WO 2022/161347

(57) **Abstract**

A series of tricyclic compounds and the use thereof. Specifically disclosed are a compound as represented by formula (II) and a pharmaceutically acceptable salt thereof.

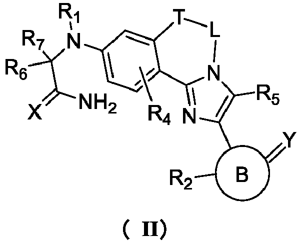

## Description

The present application claims the right of the following priorities:
CN202110134377.6, application date: January 29, 2021;
CN202210020761.8, application date: January 07, 2022.

### TECHNICAL FIELD

The present disclosure relates to a series of tricyclic compounds and a use thereof, and specifically discloses a compound of formula (II) and a pharmaceutically acceptable salt thereof.

### BACKGROUND

Phosphatidylinositol-3-kinase (PI3K) is a kind of lipid kinase composed of a regulatory subunit p85 or p101, and a catalytic subunit p110 (which is further divided into four subtypes p110a, p110b, p110g, p110d). PI3K activates the downstream Akt, etc. by catalyzing the phosphorylation of the 3'-OH of the inositol ring of phosphatidylinositol 4,5-bisphosphate (PIP2) to phosphatidylinositol 3,4,5-triphosphate (PIP3), thus playing a key role in cell proliferation, survival, and metabolism. In tumor cells, PI3K is overexpressed, leading to rapid proliferation and growth of tumor cells.

There are four subtypes of PI3K, wherein PI3Kα is widely distributed in the body. Abnormal activation of PI3Kα is also found in various solid tumors. There are also mutations in the PIK3CA gene in different solid tumors, which lead to the occurrence and development of tumors. PI3Kα mainly regulates insulin and other related blood glucose regulation pathways in normal physiological functions. Therefore, the inhibition of wild-type PI3Kα has also been clinically verified to cause side effects such as hyperglycemia. Therefore, inhibitors targeting mutant PI3Kα play an important role in clinical safety.

GDC-0077 is a highly selective PI3Kα inhibitor developed by Roche. At the same time, it has the function of degrading mutant PI3Kα protein, which brings new hope for the clinical development of PI3K inhibitors with higher safety.

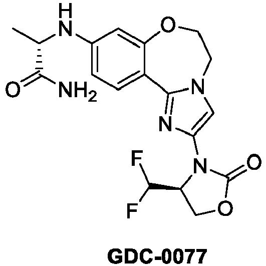

### CONTENT OF THE PRESENT INVENTION

The present disclosure provides a compound of formula (II) or a pharmaceutically acceptable salt thereof, 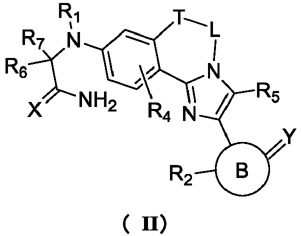 wherein
T is selected from O and S;
L is selected from -C₁₋₃ alkyl- and -C₁₋₃ alkyl-cyclopropyl-;
R₁ is selected from H and C₁₋₃ alkyl, and the C₁₋₃ alkyl is optionally substituted by 1, 2, or 3 Rₐ;
R₂ is selected from H, F, Cl, Br, I, OH, and C₁₋₃ alkyl, and the C₁₋₃ alkyl is optionally substituted by 1, 2, or 3 R_{b};
X and Y are each independently selected from O and NR₃, and X and Y are not selected from O at the same time;
R₃ is independently selected from H, OH, CN, C₁₋₃ alkyl, C₁₋₃ alkoxy, and -O-C₃₋₅ cycloalkyl, and the C₁₋₃ alkyl, C₁₋₃ alkoxy, and -O-C₃₋₅ cycloalkyl are optionally substituted by 1, 2, or 3 R_{c};
R₄ and R₅ are selected from H, F, Cl, Br, I, OH, and C₁₋₃ alkyl;
R₆ is selected from H and C₁₋₃ alkyl;
R₇ is selected from C₁₋₃ alkyl, C₁₋₃ alkoxy, and 3- to 5-membered heterocycloalkyl;
or, R₆, R₇ together with the carbon atom they share form a 3- to 5-membered heterocycloalkyl;
or, R₁, R₇ together with the atom to which they are attached form a 3 - to 5-membered heterocycloalkyl;
ring B is selected from 4- to 8-membered heterocycloalkyl, and the 4- to 8-membered heterocycloalkyl is optionally substituted by 1, 2, or 3 R_{d};
Rₐ, R_{b}, R_{c}, and R_{d} are each independently selected from F, Cl, Br, and I.

In some embodiments of the present disclosure, the above R₁ is selected from H and CH₃, and the CH₃ is optionally substituted by 1, 2, or 3 Rₐ, and other variables are as defined in the present disclosure.

In some embodiments of the present disclosure, the above R₁ is selected from H, CH₃, CH₂F, CHF₂, and CF₃, and other variables are as defined in the present disclosure.

In some embodiments of the present disclosure, the above R₂ is selected from H, F, Cl, Br, I, OH, and CH₃, and the CH₃ is optionally substituted by 1, 2, or 3 R_{b}, and other variables are as defined in the present disclosure.

In some embodiments of the present disclosure, the above R₂ is selected from H, F, Cl, Br, I, OH, CH₃, CH₂F, CHF₂, and CF₃, and other variables are as defined in the present disclosure.

In some embodiments of the present disclosure, the above R₃ is independently selected from H, OH, CN, CH₃, CH₂CH₃, OCH₃, OCH₂CH₃, -OCH(CH₃)₂, -O-cyclopropyl, and -O-cyclobutyl, and the CH₃, CH₂CH₃, OCH₃, OCH₂CH₃, -OCH(CH₃)₂, -O-cyclopropyl, and -O-cyclobutyl are optionally substituted by 1, 2, or 3 R_{c}, and other variables are as defined in the present disclosure.

In some embodiments of the present disclosure, the above R₃ is independently selected from H, OH, CN, CH₃, CH₂CH₃, OCH₃, OCH₂CH₃, -OCH(CH₃)₂, -O-cyclopropyl, and -O-cyclobutyl, and other variables are as defined in the present disclosure.

In some embodiments of the present disclosure, the above X and Y are each independently selected from O, NH, NOH, NCN, N-CH₃, N-OCH₃, N-OCH₂CH₃, N-OCH(CH₃)₂, N-O-cyclopropyl, and N-O-cyclobutyl, and other variables are as defined in the present disclosure.

In some embodiments of the present disclosure, the above R₄ and R₅ are selected from H, F, Cl, Br, I, OH, and CH₃, and other variables are as defined in the present disclosure.

In some embodiments of the present disclosure, the above R₄ is selected from H, F, Cl, Br, I, OH, and CH₃, and other variables are as defined in the present disclosure.

In some embodiments of the present disclosure, the above R₅ is selected from H, F, Cl, Br, I, OH, and CH₃, and other variables are as defined in the present disclosure.

In some embodiments of the present disclosure, the above R₇ is selected from CH₃, CH(CH₃)₂, OCH₃, and oxetanyl, and other variables are as defined in the present disclosure.

In some embodiments of the present disclosure, the above R₆, R₇ together with the carbon atom they share form an oxetanyl, and other variables are as defined in the present disclosure.

In some embodiments of the present disclosure, the above R₁, R₇ together with the atom to which they are attached form an azetidinyl and a pyrrolidinyl, and other variables are as defined in the present disclosure.

In some embodiments of the present disclosure, the above L is selected from - CH₂CH₂-, -CH(CH₃)CH₂-, and 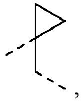 and other variables are as defined in the present disclosure.

In some embodiments of the present disclosure, the above ring B is selected from 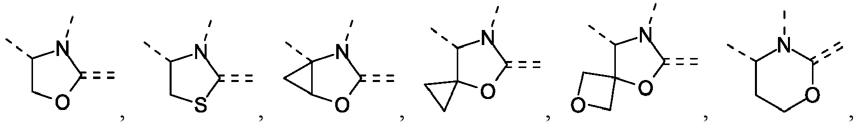 and the 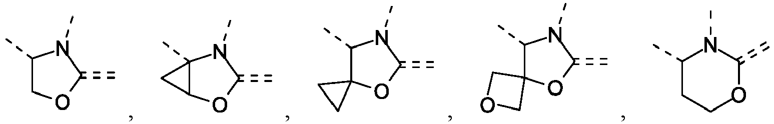 are optionally substituted by 1, 2, or 3 R_{d}, and other variables are as defined in the present disclosure.

In some embodiments of the present disclosure, the above ring B is selected from 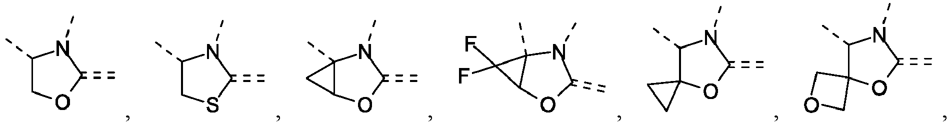 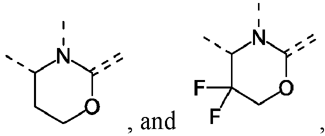 and other variables are as defined in the present disclosure.

The present disclosure provides a compound of formula (I) or a pharmaceutically acceptable salt thereof, 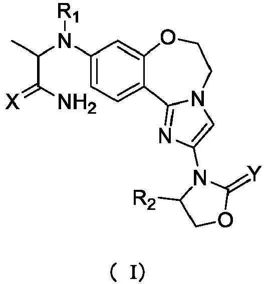 wherein
R₁ is selected from H and C₁₋₃ alkyl, and the C₁₋₃ alkyl is optionally substituted by 1, 2, or 3 Rₐ;
R₂ is selected from H, F, Cl, Br, I, OH, and C₁₋₃ alkyl, and the C₁₋₃ alkyl is optionally substituted by 1, 2, or 3 R_{b};
X and Y are each independently selected from O and NR₃, and X and Y are not selected from O at the same time;
R₃ is independently selected from H, OH, CN, C₁₋₃ alkyl, C₁₋₃ alkoxy, and -O-C₃₋₅ cycloalkyl, and the C₁₋₃ alkyl, C₁₋₃ alkoxy and -O-C₃₋₅ cycloalkyl are optionally substituted by 1, 2, or 3 R_{c};
Rₐ, R_{b}, and R_{c} are each independently selected from F, Cl, Br, and I.

In some embodiments of the present disclosure, the above R₁ is selected from H and CH₃, wherein the CH₃ is optionally substituted by 1, 2, or 3 Rₐ, and other variables are as defined in the present disclosure.

In some embodiments of the present disclosure, the above R₁ is selected from H, CH₃, CH₂F, CHF₂, and CF₃, and other variables are as defined in the present disclosure.

In some embodiments of the present disclosure, the above R₂ is selected from H, F, Cl, Br, I, OH, and CH₃, wherein the CH₃ is optionally substituted by 1, 2, or 3 R_{b}, and other variables are as defined in the present disclosure.

In some embodiments of the present disclosure, the above R₂ is selected from H, F, Cl, Br, I, OH, CH₃, CH₂F, CHF₂, and CF₃, and other variables are as defined in the present disclosure.

In some embodiments of the present disclosure, the above R₃ is independently selected from H, OH, CN, CH₃, CH₂CH₃, OCH₃, OCH₂CH₃, -OCH(CH₃)₂, -O-cyclopropyl, and -O-cyclobutyl, and the CH₃, CH₂CH₃, OCH₃, OCH₂CH₃, -OCH(CH₃)₂, -O-cyclopropyl, and -O-cyclobutyl are optionally substituted by 1, 2, or 3 R_{c}, and other variables are as defined in the present disclosure.

In some embodiments of the present disclosure, the above R₃ is independently selected from H, OH, CN, CH₃, CH₂CH₃, OCH₃, OCH₂CH₃, -OCH(CH₃)₂, -O-cyclopropyl, and -O-cyclobutyl, and other variables are as defined in the present disclosure.

In some embodiments of the present disclosure, the above X and Y are each independently selected from O, NH, NOH, NCN, N-CH₃, N-OCH₃, N-OCH₂CH₃, N-OCH(CH₃)₂, N-O-cyclopropyl, and N-O-cyclobutyl, and other variables are as defined in the present disclosure.

There are still some embodiments of the present disclosure which are obtained by any combination of the above variables.

In some embodiments of the present disclosure, the above compound or the pharmaceutically acceptable salt thereof is selected from: 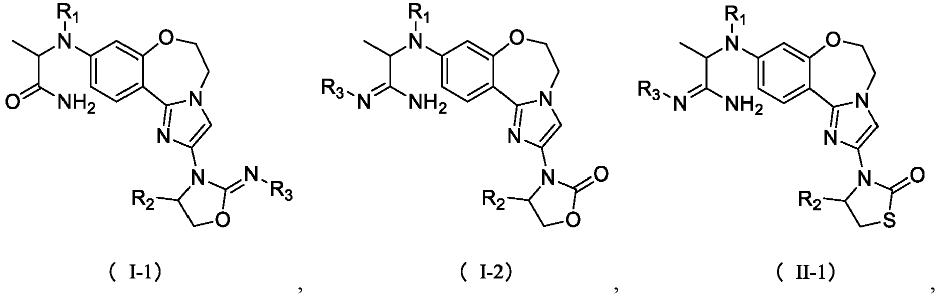 wherein
R₁, R₂, and R₃ are as defined in the present disclosure.

In some embodiments of the present disclosure, the above compound or the pharmaceutically acceptable salt thereof is selected from: 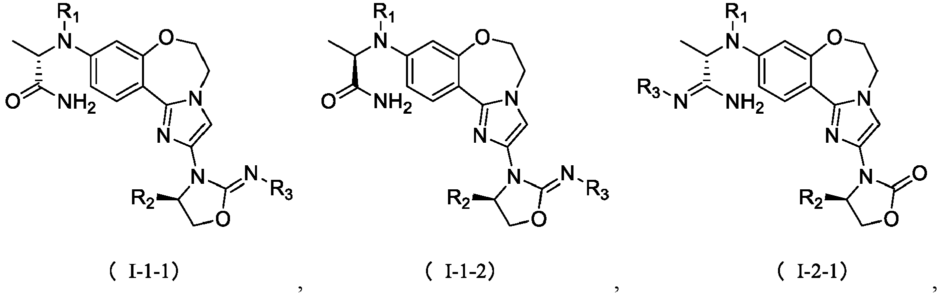 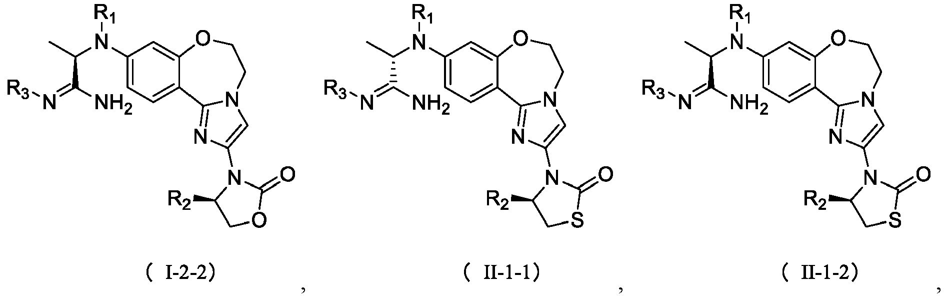 wherein
R₁, R₂, and R₃ are as defined in the present disclosure.

The present disclosure provides a compound of the following formula or a pharmaceutically acceptable salt thereof,

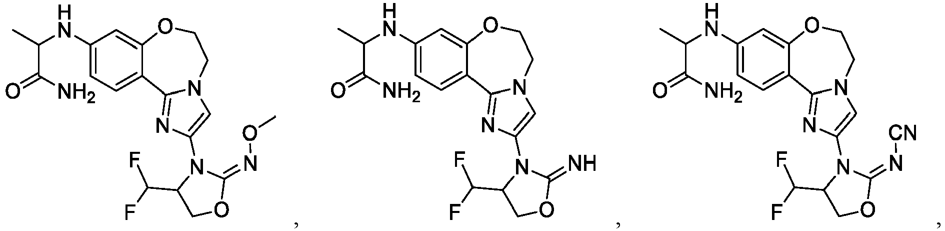

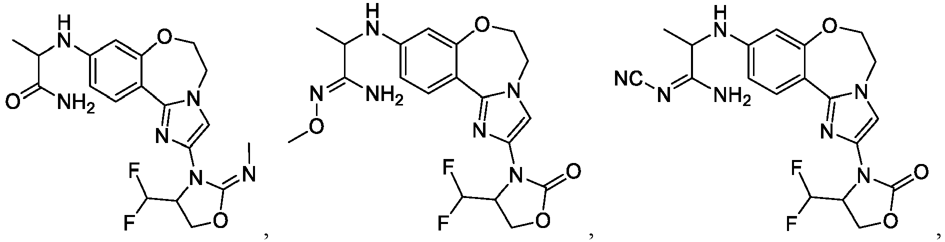

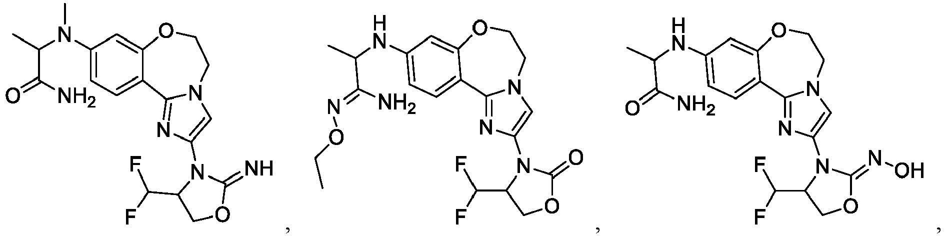

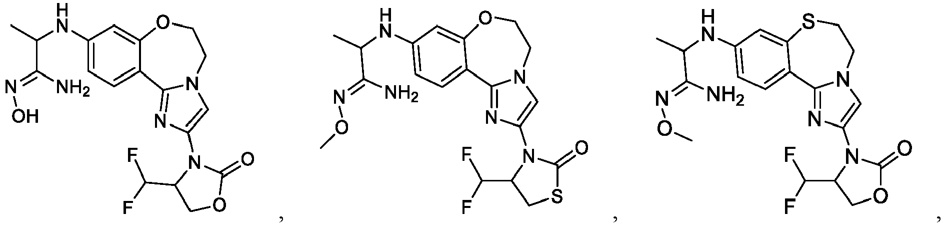

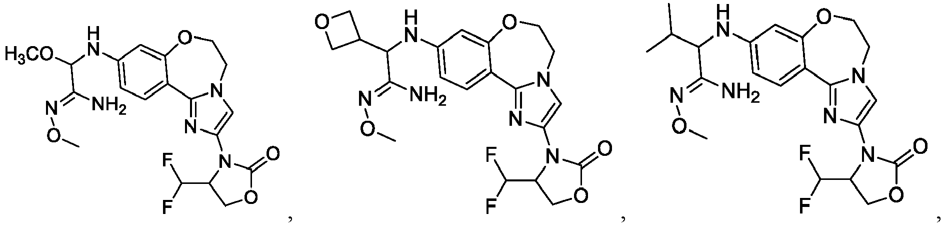

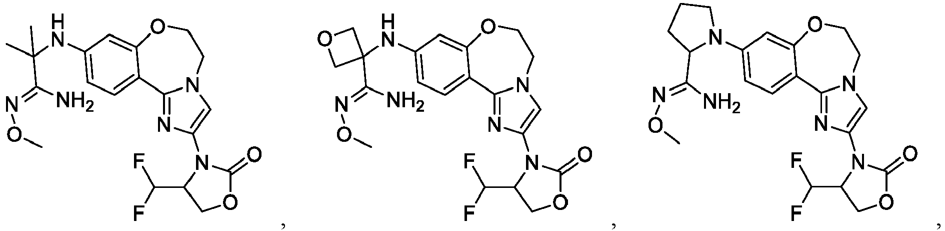

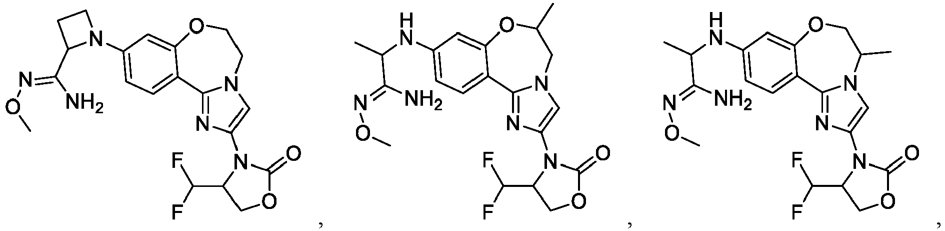

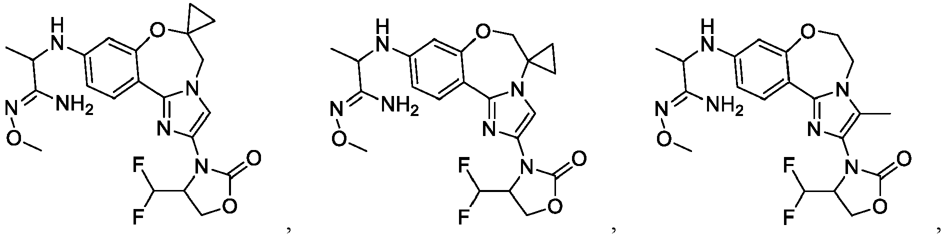

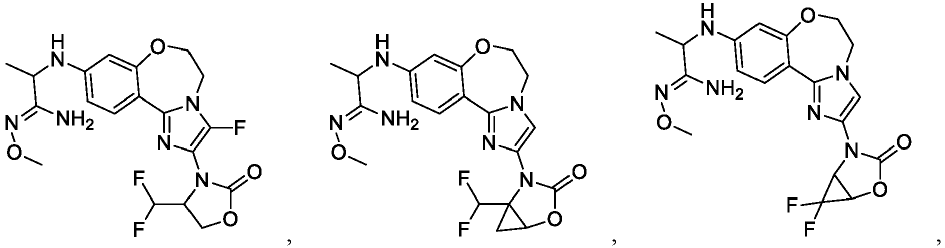

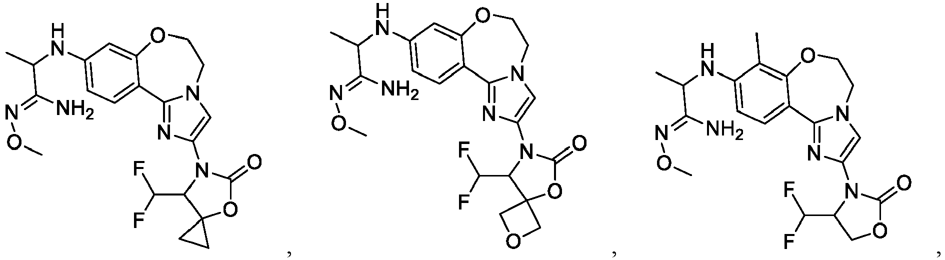

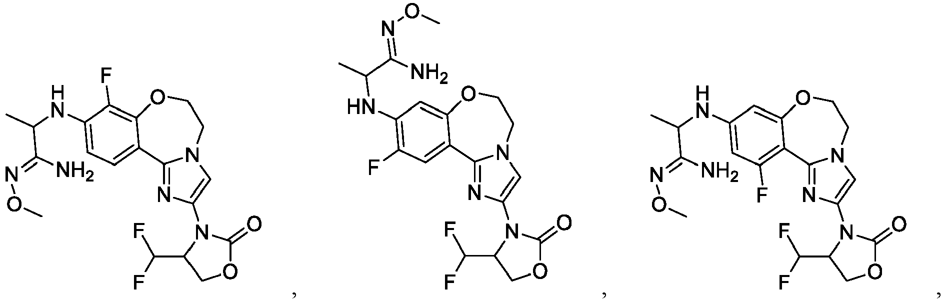

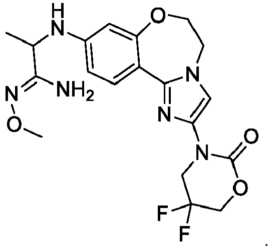

In some embodiments of the present disclosure, the above compound or the pharmaceutically acceptable salt thereof is selected from:

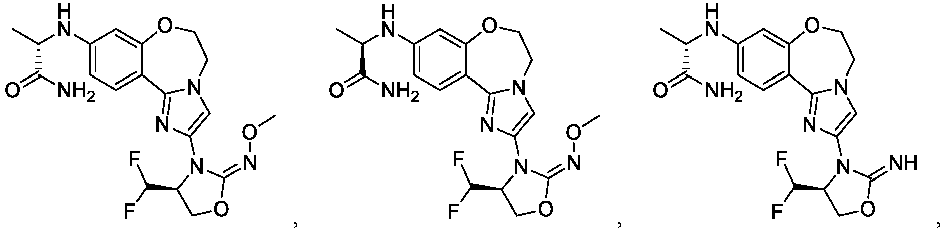

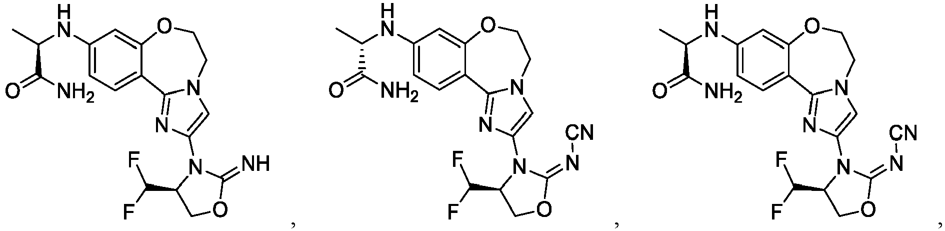

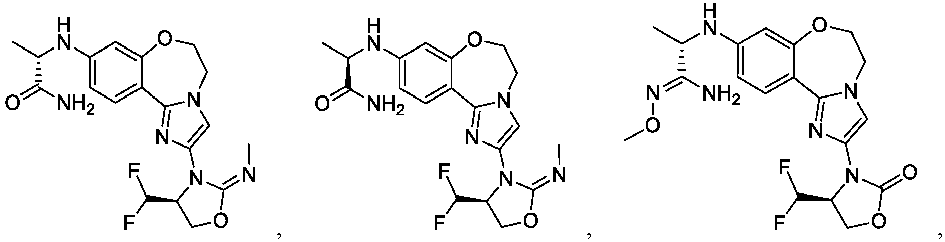

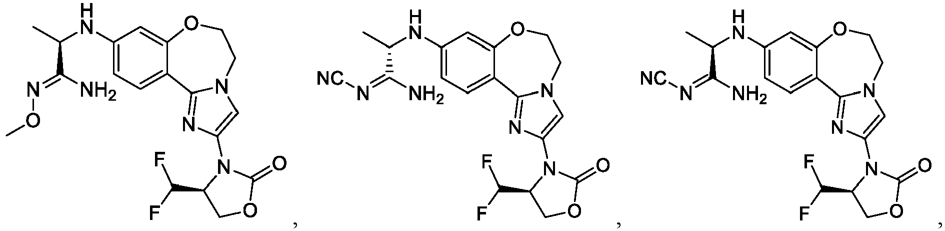

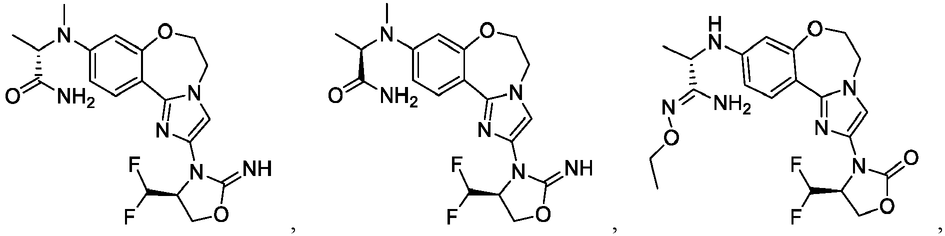

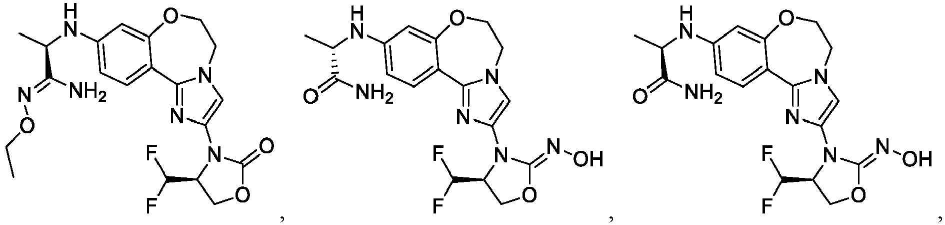

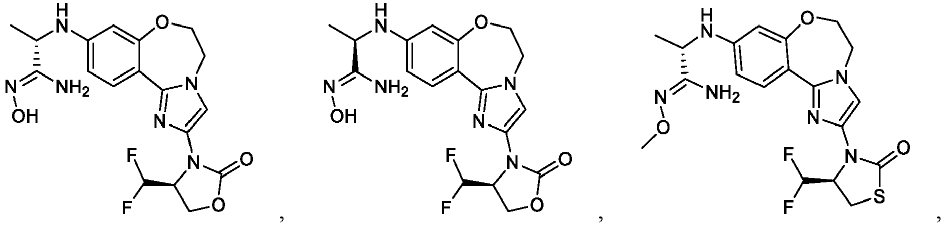

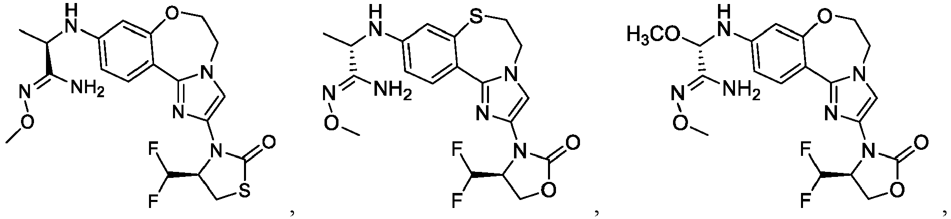

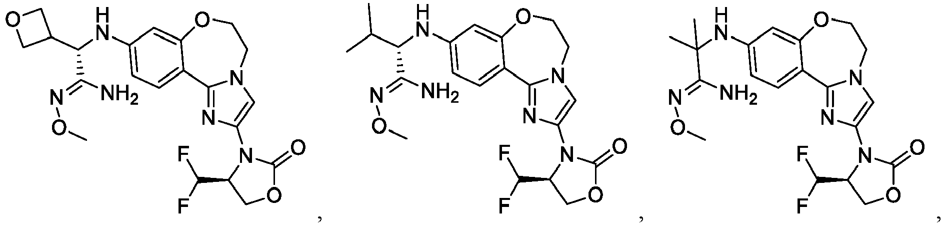

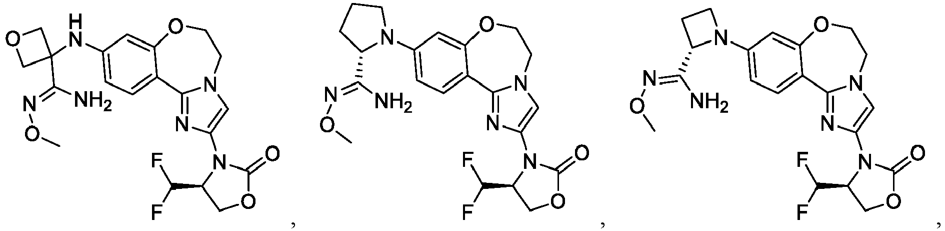

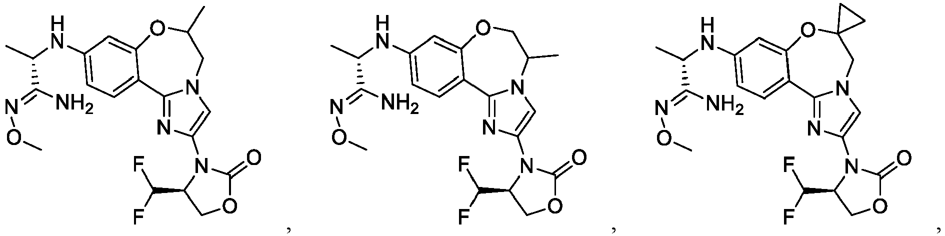

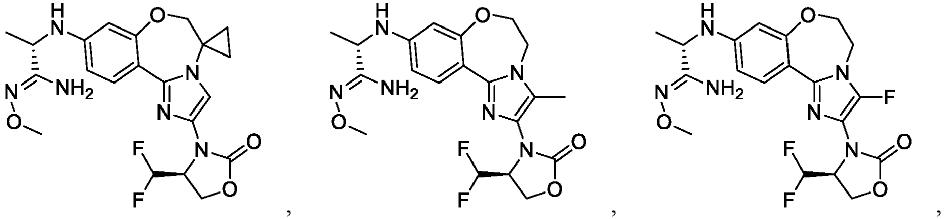

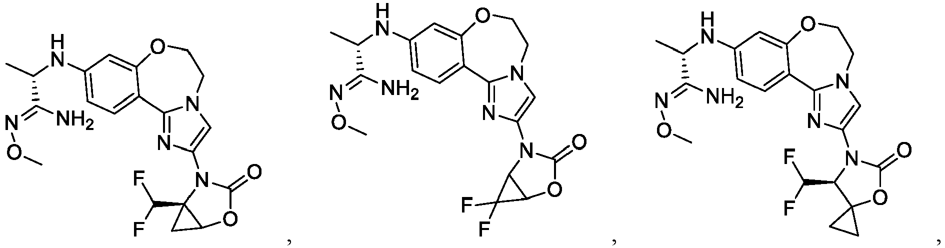

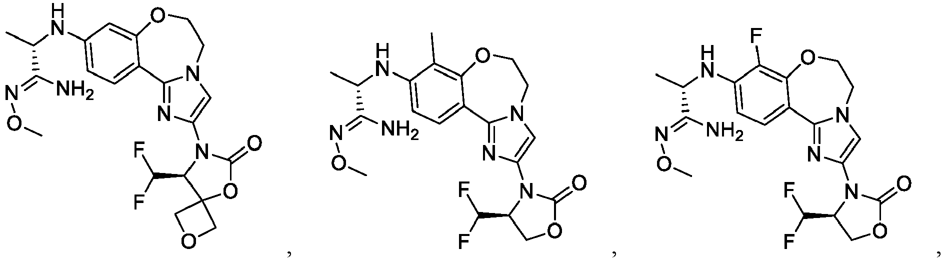

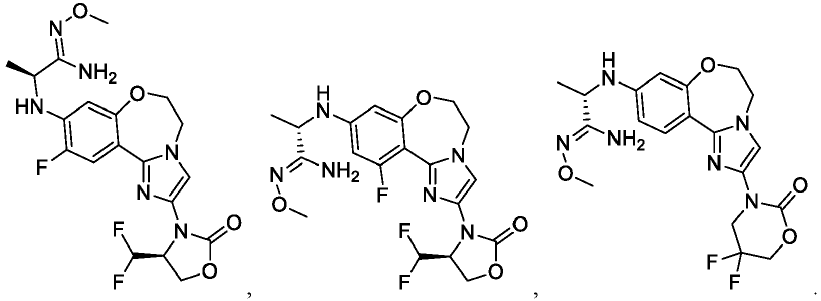

### Technical Effect

The compounds of the present disclosure can well inhibit the activity of PI3Kα kinase and have high subtype selectivity to PI3Kβ/γ/δ. In addition, the cell proliferation activity can also be well inhibited in HCC1954 cells with PIK3CA mutation; the compounds of the present disclosure have properties of high permeability and low efflux; and the compounds of the present disclosure have excellent pharmacokinetic properties.

### Definition and description

Unless otherwise specified, the following terms and phrases used herein have the following meanings. A specific term or phrase should not be considered indefinite or unclear in the absence of a particular definition, but should be understood according to the common meaning. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salt includes a salt of sodium, potassium, calcium, ammonium, organic amine, or magnesium, or similar salts. When the compound of the present disclosure contains a relatively basic functional group, an acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

Unless otherwise specified, the term "isomer" is intended to include a geometric isomer, a cis-trans isomer, a stereoisomer, an enantiomer, an optical isomer, a diastereoisomer, and a tautomeric isomer.

The compounds of the present disclosure may exist in specific geometric or stereoisomeric forms. The present disclosure contemplates all such compounds, including cis and trans isomers, (-)-and (+)-enantiomers, (*R*)-and (*S*)-enantiomers, diastereoisomers, (*D*)-isomers, (*L*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 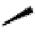 ) and a wedged dashed bond ( 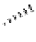 ), and the relative configuration of a stereogenic center is represented by a straight solid bond ( 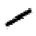 ) and a straight dashed bond ( 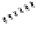 ), a wave line ( 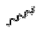 ) is used to represent a wedged solid bond ( 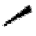 ) or a wedged dashed bond ( 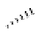 ), or the wave line ( 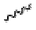 ) is used to represent a straight solid bond ( 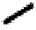 ) or a straight dashed bond ( 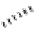 ).

Unless otherwise specified, when double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by a wave line ( 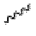 ), this refers to the (Z) isomer, (E) isomer, or a mixture of two isomers of the compound. For example, the following formula (A) means that the compound exists as a single isomer of formula (A-1) or formula (A-2) or as a mixture of two isomers of formula (A-1) and formula (A-2); the following formula (B) means that the compound exists in the form of a single isomer of formula (B-1) or formula (B-2) or in the form of a mixture of two isomers of formula (B-1) and formula (B-2). The following formula (C) means that the compound exists as a single isomer of formula (C-1) or formula (C-2) or as two a mixture of two isomers of formula (C-1) and formula (C-2).

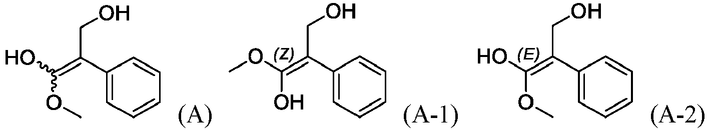

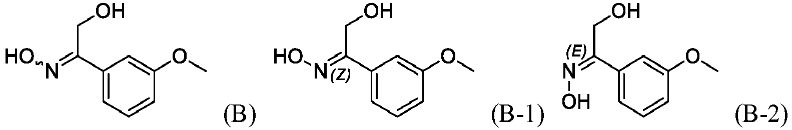

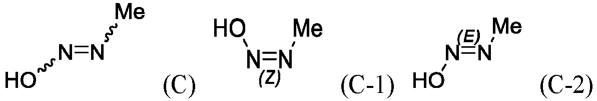

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom(s) on a specific atom are substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R groups, and the group can be optionally substituted by up to two R groups, wherein the definition of R at each occurrence is independent. Moreover, a combination of the substituent and/or the variant thereof is allowed only when the combination results in a stable compound.

When the enumerative linking group does not indicate the direction for linking, the direction for linking is arbitrary, for example, the linking group L contained in 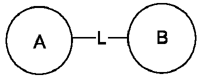 is -M-W-, then -M-W- can link ring A and ring B to form 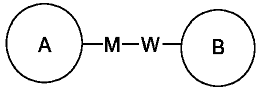 in the direction same as left-to-right reading order, and form 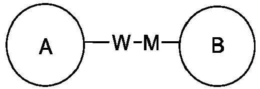 in the direction contrary to left-to-right reading order. A combination of the linking groups, substituents, and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atoms at the site will decrease correspondingly with the number of chemical bonds linking thereto so as to meet the corresponding valence. The chemical bond between the site and other groups can be represented by a straight solid bond ( 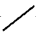 ), a straight dashed bond ( 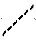 ), or a wavy line ( 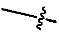 ). For example, the straight solid bond in -OCH₃ means that it is linked to other groups through the oxygen atom in the group; the straight dashed bond in 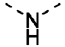 means that it is linked to other groups through the two ends of the nitrogen atom in the group; the wave lines in 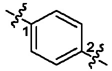 means that the phenyl group is linked to other groups through carbon atoms at position 1 and position 2; 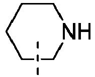 means that it can be linked to other groups through any linkable sites on the piperidinyl by one chemical bond, including at least four types of linkage, including 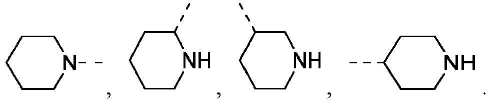 Even though the H atom is drawn on the -N-, 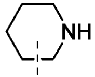 still includes the linkage of 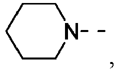 merely when one chemical bond was connected, the H of this site will be reduced by one to the corresponding monovalent piperidinyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, and C₂ alkoxy, etc. Examples of C₁₋₃ alkoxy include, but are not limited to, methoxy, ethoxy, propoxy (including *n*-propoxy and isopropoxy), etc.

Unless otherwise specified, "C₃₋₅ cycloalkyl" refers to a saturated cyclic hydrocarbon group consisting of 3 to 5 carbon atoms, which is a monocyclic system, and the C₃₋₅ cycloalkyl includes C₃₋₄ and C₄₋₅ cycloalkyl, etc.; it can be monovalent, divalent, or polyvalent. Examples of C₃₋₅ cycloalkyl include, but are not limited to, cyclopropyl, cyclobutyl, cyclopentyl, etc.

Unless otherwise specified, the term "3- to 5-membered heterocycloalkyl" by itself or in combination with other terms refers to a saturated monocyclic group consisting of 3 to 5 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). In addition, with regard to the "3- to 5-membered heterocycloalkyl", a heteroatom may occupy the connection position of the heterocycloalkyl with the rest of the molecule. The 3- to 5-membered heterocycloalkyl includes 4- to 5-membered, 4-membered, and 5-membered heterocycloalkyl, etc. Examples of 3- to 5-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl and tetrahydrothiophen-3-yl, etc.), or tetrahydrofuranyl (including tetrahydrofuran-2-yl, etc.) and the like.

Unless otherwise specified, the term "4- to 8-membered heterocycloalkyl" by itself or in combination with other terms refers to a saturated cyclic group consisting of 4 to 8 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic systems include spiro ring, fused ring and bridged ring. In addition, with regard to the "4- to 8-membered heterocycloalkyl", a heteroatom may occupy the connection position of the heterocycloalkyl with the rest of the molecule. The 4- to 8-membered heterocycloalkyl includes 4- to 6-membered, 4- to 5-membered, 5- to 6-membered, 4-membered, 5-membered, and 6-membered heterocycloalkyl, etc. Examples of 4- to 8-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxinyl, dithianyl, isoxazolidinyl, isothiazolidinyl, 1,2-oxazinyl, 1,2-thiazinyl, hexahydropyridazinyl, homopiperazinyl, homopiperidinyl, or dioxacycloheptyl, etc.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art. Preferred embodiments include, but are not limited to, the examples of the present disclosure.

The solvents used in the present disclosure are commercially available.

The present disclosure adopts the following abbreviations: aq represents water; eq represents equivalent; DCM represents dichloromethane; PE represents petroleum ether; DMSO represents dimethyl sulfoxide; EtOAc represents ethyl acetate; EtOH represents ethanol; MeOH represents methanol; DMF represents N,N-dimethylformamide; Cbz represents benzyloxycarbonyl, which is an amine protecting group; Boc represents tert-butoxycarbonyl, which is an amine protecting group; r.t. represents room temperature; O/N represents overnight; THF represents tetrahydrofuran; Boc₂O represents di-tert-butyl dicarbonate; TFA represents trifluoroacetic acid; HCl represents hydrochloric acid; iPrOH represents 2-propanol; mp represents melting point; Pd(PPh₃)₄ represents tetrakis(triphenylphosphine)palladium; Pd(dppf)Cl₂ represents [1,1'-bis(diphenylphosphino)ferrocene]dichloropalladium(II); DIBAL-H represents diisobutylaluminum hydride; NIS represents N-iodosuccinimide; Dess-Martin represents Dess Martin; BAST represents bis(2-methoxy)ethyl sulfur trifluoride; HATU represents O-(7-azabenzotriazol-1-yl)-N,N,N',N'-tetramethyluronium hexafluorophosphate; HOSu represents N-hydroxysuccinimide; EDCI represents N-(3-dimethylaminopropyl)-N'-ethylcarbodiimide hydrochloride.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and specific embodiments thereof have also been disclosed; for those skilled in the art, it is obvious to make various modifications and improvements to the embodiments of the present disclosure without departing from the spirit and scope of the present disclosure.

### Reference example 1: Moiety BB-1

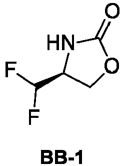

### Synthetic Route:

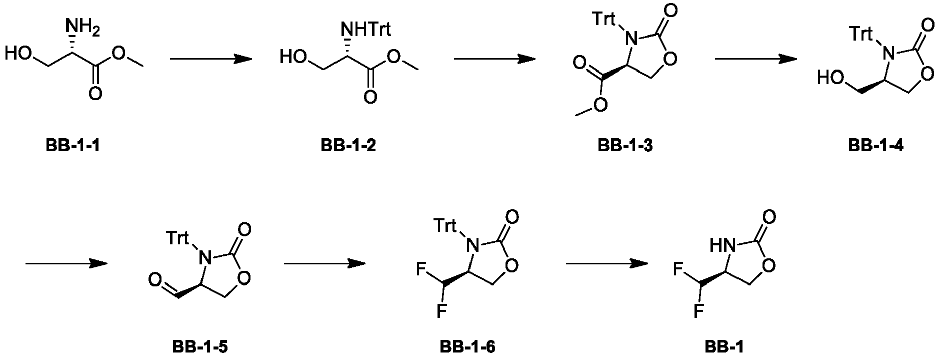

### Step 1: Synthesis of Compound BB-1-2

**BB-1-1** (50 g, 321.38 mmol, 1 eq, HCl) was dissolved in dichloromethane (500 mL). Triethylamine (65.04 g, 642.76 mmol, 89.46 mL, 2 eq) was added thereto. The system was replaced with nitrogen, and then the mixture was cooled to 0°C, and then a solution of triphenylchloromethane (89.59 g, 321.38 mmol, 1 eq) in dichloromethane (300 mL) was added dropwise thereto. The reaction mixture was slowly warmed to 20°C and stirred for 10 hours. After the reaction was completed, the reaction mixture was poured into saturated sodium chloride (200 mL) and quenched slowly at 0°C, then extracted with dichloromethane (200 mL * 3). The organic phases were combined, washed with saturated sodium chloride (100 mL), then dried over anhydrous sodium sulfate, filtered, and finally evaporated to dryness by rotary evaporation under reduced pressure to obtain compound **BB-1-2,** which was directly used in the next reaction step. ¹H NMR (400 MHz, CDCl₃) δ 7.41 (d, *J* = 7.5 Hz, 6H), 7.22 - 7.17 (m, 6H), 7.16 - 7.08 (m, 3H), 3.62 (br d, *J* = 3.9 Hz, 1H), 3.54 - 3.43 (m, 2H), 3.22 (s, 3H).

### Step 2: Synthesis of Compound BB-1-3

Compound **BB-1-2** (55 g, 152.17 mmol, 1 *eq*)*,* toluene (390 mL), triethylamine (39.57 g, 391.08 mmol, 54.43 mL, 2.57 *eq*) were added to a dry reaction flask. After the system was replaced with nitrogen, the mixture was cooled to 0°C, and a solution of triphosgene (76.77 g, 258.69 mmol, 1.7 *eq*) in toluene (165 mL) was slowly added thereto. After the system was replaced with nitrogen, the mixture was stirred at 25°C for 16 hours. After the reaction was completed, 600 mL of saturated sodium carbonate solution was slowly added to the reaction mixture at 0°C to quench. The mixture was extracted with ethyl acetate (50 mL * 3). The organic phases were combined, washed with saturated brine (50 mL) in sequence, and dried over anhydrous sodium sulfate. The organic phases were filtered and evaporated to dryness by rotary evaporation under reduced pressure to obtain a crude product, and then the crude product was slurried with 400 mL of a mixed solution (petroleum ether: ethyl acetate = 3:1) for 0.5 hours, filtered, and the filter cake was evaporated to dryness by rotary evaporation under reduced pressure to obtain compound **BB-1-3.** ¹H NMR (400MHz, CDCl₃) δ (ppm) 7.26-7.40 (m, 15H), 4.51-4.63 (m, 1H), 4.41-4.50 (m, 1H), 4.21 (dd, *J*=8.8, 3.2 Hz, 1H), 3.49 (s, 3H).

### Step 3: Synthesis of Compound BB-1-4

**BB-1-3** (45 g, 116.15 mmol, 1 *eq*) was dissolved in tetrahydrofuran (450 mL). After the system was replaced with nitrogen, the mixture was cooled to -30°C, and lithium aluminum tetrahydride (5.29 g, 139.38 mmol, 1.2 *eq*) was slowly added thereto. The reaction mixture was stirred at -30°C for 2 hours. Two pots were fed with the same amount of reactants in parallel. After the reaction was completed, the reaction mixture was warmed to -10 to 0°C and slowly quenched with ethyl acetate (5.3 mL). Then water (5.3 mL), 20% sodium hydroxide (5.3 mL), and water (21.2 mL) were added thereto in sequence. After the mixture was stirred for 0.5 hours, anhydrous magnesium sulfate (10.6 g) was added thereto. The mixture was stirred for 0.5 hours and filtered, and the filter cake was washed with ethyl acetate (500 mL). The filtrates were combined and concentrated to obtain compound **BB-1-4,** which was directly used in the next reaction step without purification. ¹H NMR (400 MHz, CDCl₃) δ 7.39 - 7.28 (m, 15H), 4.50 - 4.29 (m, 2H), 3.89 - 3.77 (m, 1H), 3.43 - 3.31 (m, 1H), 3.30 - 3.18 (m, 1H).

### Step 4: Synthesis of Compound BB-1-5

Compound **BB-1-4** (30 g, 83.47 mmol, 1 *eq*)*,* Dess-Martin (42.48 g, 100.16 mmol, 31.01 mL, 1.2 *eq*)*,* and dichloromethane (600 mL) were added to a dry reaction flask. After the system was replaced with nitrogen, the reaction was stirred at 20°C for 16 hours. After the reaction was completed, the reaction mixture was added with saturated sodium thiosulfate solution (300 mL), stirred for 1 hour, and then extracted with dichloromethane (300 mL * 2). The organic phases were combined, washed with saturated sodium carbonate (300 mL * 2)and saturated brine (300 mL) in sequence, dried over anhydrous sodium sulfate, and filtered. The crude product was slurried with 500 mL of petroleum ether, filtered, and the filter cake was evaporated to dryness by rotary evaporation to obtain compound **BB-1-5.** ¹H NMR (400MHz, CDCl₃) δ (ppm) 9.24 (d, *J*=3.1 Hz, 1H), 7.32-7.36 (m, 15H), 4.49-4.55 (m, 1H), 4.38 (dt, *J*=9.6, 3.8 Hz, 1H), 4.23 (dd, *J*=9.3, 4.5 Hz, 1H).

### Step 5: Synthesis of Compound BB-1-6

**BB-1-5** (17 g, 47.57 mmol, 1 *eq*) was dissolved in dichloromethane (170 mL). After the system was replaced with nitrogen, the mixture was cooled to 0°C, and BAST (26.31 g, 118.91 mmol, 26.05 mL, 2.5 *eq*) was slowly added thereto. The reaction mixture was slowly warmed to 20°C and stirred for 10 hours, then warmed to 35°C and stirred for 2 hours. Two pots were fed with the same amount of reactants in parallel. After the reaction was completed, the reaction mixtures were combined and quenched slowly with saturated sodium bicarbonate (500 mL) at 0 to 10°C, then extracted with dichloromethane (200 mL * 3). The organic phases were combined, washed with saturated sodium chloride (100 mL) and dried over anhydrous sodium sulfate. The organic phase was filtrated, and evaporated to dryness by rotary evaporation under reduced pressure, and the crude product was separated by silica gel column chromatography (silica gel mesh: 100 to 200 mesh; petroleum ether: ethyl acetate = 5:1 to 1:1) to obtain compound **BB-1-6.** ¹H NMR (400 MHz, CDCl₃) δ 7.42 - 7.34 (m, 9H), 7.33 - 7.28 (m, 6H), 4.91 - 4.56 (m, 2H), 4.46 (t, *J* = 9.2 Hz, 1H), 4.21 - 4.06 (m, 1H).

### Step 6: Synthesis of Compound BB-1

**BB-1-6** (8 g, 21.09 mmol, 1 *eq*) was dissolved in methanol hydrochloride (4 M, 160.00 mL, 30.35 *eq*) and methanol (2 mL). The reaction mixture was heated to 50°C and stirred for 10 hours after the system was replaced with nitrogen. After the reaction was completed, the reaction mixture was cooled to 20°C, and then evaporated to dryness by rotary evaporation. The crude product was separated by silica gel column chromatography (silica gel mesh: 100 to 200 mesh; dichloromethane: methanol = 100:0.1 to 100:2) to obtain compound **BB-1.** ¹H NMR (400 MHz, CDCl₃) δ 6.07 (br dd, *J* = 5.3, 6.7 Hz, 1H), 5.94 - 5.60 (m, 1H), 4.59 - 4.51 (m, 1H), 4.43 (dd, *J* = 4.1, 9.5 Hz, 1H), 4.12 (tt, *J* = 4.4, 8.9 Hz, 1H).

### Reference example 2: Moiety BB-2

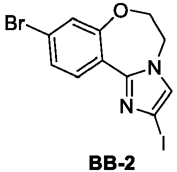

### Synthetic Route:

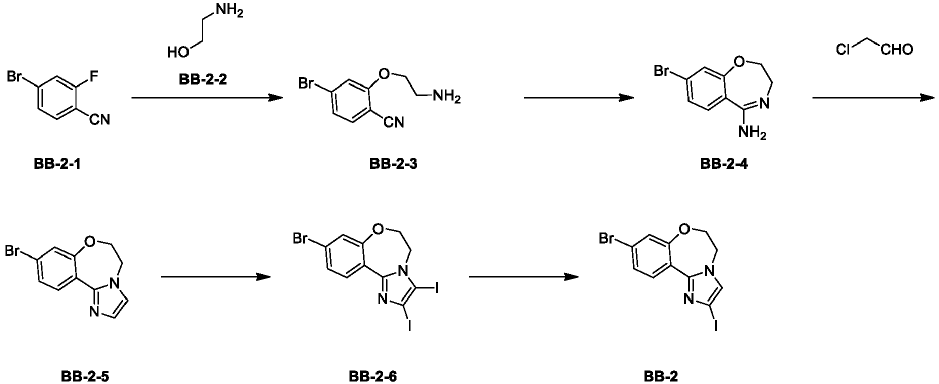

### Step 1: Synthesis of Compound BB-2-3

Compound **BB-2-2** (23.52 g, 384.99 mmol, 23.28 mL, 1.1 *eq*) was added to a dry reaction flask. After tetrahydrofuran (70 mL) was added thereto and dissolved completely, potassium tert-butoxide (47.13 g, 419.98 mmol, 1.2 *eq*) was added thereto at 5°C. After the system was replaced with nitrogen, and the reaction mixture was stirred and reacted for 40 minutes, a solution of tetrahydrofuran (210 mL) containing compound **BB-2-1** (70 g, 349.99 mmol, 1 *eq*) was added thereto. After the system was replaced with nitrogen, the reaction mixture was stirred at 5°C for 16 hours. After the reaction was completed, the reaction mixture was quenched by adding 200 mL of water, extracted with ethyl acetate (250 mL * 2). The organic phases were combined, added with saturated brine (250 mL) in sequence, dried over anhydrous sodium sulfate, and evaporated to dryness by rotary evaporation under reduced pressure to obtain the crude product. The crude product was dissolved in 280 mL of tetrahydrofuran, then 3 mol/L propanol hydrochloride (180 mL) (self-made) was added thereto. The mixture was stirred at 70°C for 3 hours, cooled to room temperature naturally, filtered, and evaporated to dryness by rotary evaporation under reduced pressure to obtain a hydrochloride of compound **BB-2-3.** ¹H NMR (400MHz, DMSO-*d*₆) δ (ppm) 8.41 (br s, 3H), 7.72 (d, *J*=8.3 Hz, 1H), 7.60 (d, *J*=1.4 Hz, 1H), 7.36 (dd, *J*=8.3, 1.4 Hz, 1H), 4.44 (t, *J*=5.1 Hz, 2H), 3.22 (br d, *J*=4.5 Hz, 2H).

### Step 2: Synthesis of Compound BB-2-4

Compound **BB-2-3** (80 g, 288.24 mmol, 1 *eq,* HCl), methanol (280 mL), diethoxymagnesium (79.16 g, 691.78 mmol, 2.4 *eq*)*,* and 2- methyltetrahydrofuran (640 mL) were added to a dry reaction flask. The system was replaced with nitrogen, and then the reaction was stirred at 70°C for 60 hours. After the reaction was completed, when the reaction was cooled to room temperature, about 300 mL of liquid in the reaction mixture was evaporated by rotary evaporation under reduced pressure at 40°C. Then 2-methyltetrahydrofuran (700 mL) and 3mol/L propanol hydrochloride solution (560 mL) were added to the remaining reaction mixture. The reaction mixture was stirred at room temperature for 3 hours and then filtered. The filter cake was rinsed with (100 mL) 2-methyltetrahydrofuran, and the rinsed filter cake was evaporated to dryness by rotary evaporation under reduced pressure to obtain a hydrochloride of compound **BB-2-4.** ¹H NMR (400MHz, DMSO-*d*₆) δ (ppm) 10.49 (br s, 1H), 9.49-9.71 (m, 2H), 7.59-7.68 (m, 2H), 7.50 (d, *J*=1.5 Hz, 1H), 4.45 (t, *J*=5.3 Hz, 2H), 3.52 (q, *J*=4.9 Hz, 2H).

### Step 3: Synthesis of Compound BB-2-5

Compound **BB-2-4** (50 g, 180.15 mmol, 1 *eq,* HCl), 2-methyltetrahydrofuran (400 mL), chloroacetaldehyde (45.96 g, 234.20 mmol, 37.67 mL, purity of 40%, 1.3 *eq*)*,* and water (25 mL) were added to a dry reaction flask. After the system was replaced with nitrogen, the mixture was warmed to 40°C, and added with saturated potassium bicarbonate solution (3.37 M, 267.29 mL, 5 *eq*)*.* The reaction mixture was heated to 45°C after the system was replaced with nitrogen, and the reaction mixture was stirred for 16 hours. After the reaction was completed, when the reaction was cooled to room temperature, the reaction mixture was washed by adding saturated sodium bisulfite solution, then added with saturated sodium carbonate solution to adjust the pH to 9 to 10, extracted with ethyl acetate (400 mL * 3). The organic phases were combined, washed with saturated brine (500 mL) in sequence, dried over anhydrous sodium sulfate, and evaporated to dryness by rotary evaporation under reduced pressure to obtain compound **BB-2-5.** ¹H NMR (400MHz, DMSO-*d*₆) δ (ppm) 8.32 (d, *J*=8.6 Hz, 1H), 7.33 (s, 1H), 7.22-7.28 (m, 2H), 7.06 (d, *J*=0.9 Hz, 1H), 4.42-4.46 (m, 4H).

### Step 4: Synthesis of Compound BB-2-6

Compound **BB-2-5** (50 g, 188.60 mmol, 1 *eq*)*,* DMF (250 mL), and NIS (91.23 g, 405.50 mmol, 2.15 *eq*) were added to a dry reaction flask. After the system was replaced with nitrogen, the reaction mixture was heated to 70°C, and stirred for 16 hours. After the reaction was completed, the reaction mixture was quenched by slowly adding (200 mL) 5% glacial acetic acid solution. Dichloromethane (250 mL * 3) was added to the quenched reaction mixture. The organic phases were combined, and then washed with saturated brine (250 mL) in sequence, and dried over anhydrous sodium sulfate. The organic phases were filtered, and the filtrate was evaporated to dryness by rotary evaporation under reduced pressure to obtain the crude product. The crude product was slurried with methyl tert-butyl ether (500 mL) for 0.5 hours and filtered. The filter cake was rinsed with methyl tert-butyl ether (300 mL) and evaporated to dryness by rotary evaporation under reduced pressure to obtain compound **BB-2-6.** ¹H NMR (400MHz, DMSO-*d*₆) δ (ppm) 8.20 (d, *J*=8.6 Hz, 1H), 7.16-7.36 (m, 2H), 4.42-4.54 (m, 2H), 4.31-4.40 (m, 2H).

### Step 5: Synthesis of Compound BB-2

Compound **BB-2-6** (52 g, 100.60 mmol, 1 *eq*) and tetrahydrofuran (25 mL) were added to the dry reaction flask. After the system was replaced with nitrogen, the mixture was cooled to 10°C, slowly added with ethylmagnesium bromide (3 M, 40.24 mL, 1.2 *eq*)*.* After the system was replaced with nitrogen again, the reaction was stirred at 10°C for 2 hours. After the reaction was completed, the reaction mixture was quenched by slowly adding 5% glacial acetic acid solution (200 mL). Ethyl acetate (250 mL * 3) was then added to the quenched reaction mixture. The organic phases were combined, washed with saturated brine (250 mL) in sequence, dried over anhydrous sodium sulfate, and filtered. The filtrate was evaporated to dryness by rotary evaporation under reduced pressure to obtain the crude product. The crude product was separated by silica gel column chromatography (petroleum ether: ethyl acetate = 1:0 to 3:1) with gradient elution to obtain compound **BB-2.** ¹H NMR (400MHz, DMSO-*d*₆) δ (ppm) 8.21 (d, *J*=8.6 Hz, 1H), 7.54 (s, 1H), 7.23-7.30 (m, 2H), 4.40-4.46 (m, 4H).

### Example 1

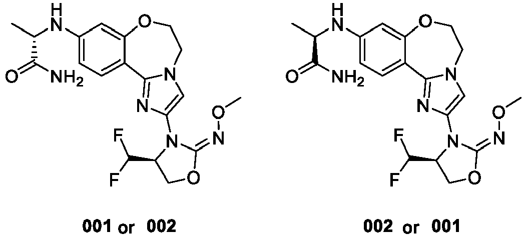

### Synthetic Route:

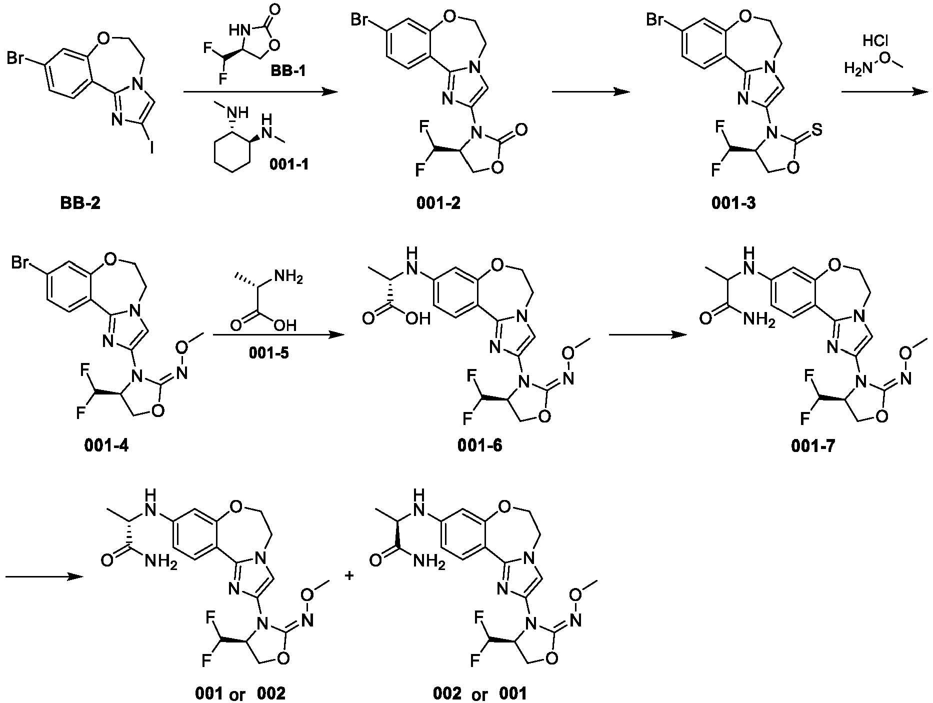

### Step 1: Synthesis of Compound 001-2

**BB-1** (1.6 g, 11.67 mmol, 1 *eq*)*,* copper acetate hydrate (2.10 g, 10.50 mmol, 2.10 mL, 0.9 *eq*)*,* **BB-2** (4.56 g, 11.67 mmol, 1 *eq*)*,* cesium carbonate (7.23 g, 22.18 mmol, 1.9 *eq*)*,* **001-1** (664.08 mg, 4.67 mmol, 0.4 *eq),* and dioxane (40 mL) were added to a dry reaction flask. After the system was replaced with nitrogen, the reaction was stirred at 110°C for 5 hours. After the reaction was completed, the reaction mixture was cooled to 20°C and evaporated to dryness by rotary evaporation under reduced pressure. The crude product was separated by silica gel column chromatography (silica gel mesh: 100 to 200 mesh; petroleum ether: ethyl acetate = 10:1 to 5:1) to obtain compound **001-2.** ¹H NMR (400 MHz, CDCl₃) δ 8.21 (d, *J* = 9.2 Hz, 1H), 7.30 (s, 1H), 7.25 - 7.16 (m, 2H), 6.86 - 6.48 (m, 1H), 4.97 - 4.82 (m, 1H), 4.74 (dd, *J* = 4.0, 9.4 Hz, 1H), 4.61 - 4.51 (m, 1H), 4.49 - 4.42 (m, 2H), 4.39 - 4.31 (m, 2H).

### Step 2: Synthesis of Compound 001-3

**001-2** (1 g, 2.50 mmol, 1 *eq*)*,* Lawesson's reagent (5.05 g, 12.49 mmol, 5 *eq*)*,* and toluene (50 mL) were added to a dry reaction flask. After the system was replaced with nitrogen, the reaction was stirred at 130°C for 10 hours. After the reaction was completed, the reaction mixture was cooled, and evaporated by rotary evaporation under reduced pressure. The crude product was separated by silica gel column chromatography (silica gel mesh: 100 to 200 mesh; mobile phase petroleum ether: mobile phase ethyl acetate = 100:1 to 20:1) to obtain compound **001-3.** ¹H NMR (400 MHz, CDCl₃) δ 7.33 (d, *J* = 9.2 Hz, 1H), 6.61 - 6.26 (m, 3H), 5.94 - 5.57 (m, 1H), 4.44 - 4.30 (m, 1H), 4.09 (dd, *J* = 3.9, 9.7 Hz, 1H), 3.84 (t, *J* = 9.6 Hz, 1H), 3.68 - 3.59 (m, 2H), 3.55 (br dd, *J* = 3.0, 4.9 Hz, 2H).

### Step 3: Synthesis of Compound 001-4

**001-3** (2.8 g, 6.73 mmol, 1 *eq*)*,* O-methylhydroxylamine hydrochloride (1.80 g, 21.53 mmol, 3.2 *eq*)*,* triethylamine (4.08 g, 40.36 mmol, 5.62 mL, 6 *eq*)*,* and mercuric oxide (14.57 g, 67.27 mmol, 10 *eq*) were added to a dry reaction flask. Then DMF (56 mL) was added thereto. The mixture was heated to 60°C and stirred for 10 hours after the system was replaced with nitrogen. After the reaction was completed, the reaction mixture was diluted with dichloromethane (100 mL) and filtered. The filtrate was extracted with ethyl acetate (100 mL * 3). The organic phases were combined, washed with saturated sodium chloride (10 mL), then dried over anhydrous sodium sulfate, filtered and finally evaporated to dryness by rotary evaporation under reduced pressure. The crude product was separated by silica gel column chromatography (silica gel mesh: 100 to 200 mesh; petroleum ether: ethyl acetate = 5:1 to 3:1) to obtain compound **001-4.** ¹H NMR (400 MHz, CDCl₃) δ 8.41 - 7.96 (m, 1H), 7.47 - 7.38 (m, 1H), 7.25 - 7.16 (m, 2H), 6.80 - 6.38 (m, 1H), 5.03 - 4.87 (m, 1H), 4.82 (dd, *J* = 3.5, 9.2 Hz, 1H), 4.64 - 4.54 (m, 1H), 4.50 - 4.41 (m, 2H), 4.39 - 4.32 (m, 2H), 3.82 (s, 3H).

### Step 4: Synthesis of Compound 001-6

**001-4** (0.5 g, 1.16 mmol, 1 *eq*)*,* **001-5** (415.14 mg, 4.66 mmol, 4 *eq*)*,* and potassium phosphate (1.24 g, 5.82 mmol, 5 *eq*) were dissolved in DMSO (11 mL). After the system was replaced with nitrogen, copper iodide (66.56 mg, 349.47 µmol, 0.3 *eq*) was added thereto. The reaction mixture was heated to 120°C under microwave irradiation and stirred for 1.5 hours. After the reaction was completed, the reaction mixture was cooled to 20°C and filtered, and the filtrate was separated by preparative high performance liquid chromatography (chromatographic column: Waters Xbridge Prep OBD C18 150 * 40 mm * 10 µm; mobile phase: [water (10 mM NH₄HCO₃)-ACN]; acetonitrile: 8% to 38%, 8 min) to obtain compound **001-6.** ¹HNMR (400 MHz, CDCl₃) δ 8.13 (br d, *J* = 8.6 Hz, 1H), 7.15 - 6.95 (m, 1H), 6.82 - 6.04 (m, 3H), 5.08 - 4.84 (m, 1H), 4.80 (dd, *J =* 3.2, 9.4 Hz, 1H), 4.65 - 4.51 (m, 1H), 4.41 (br s, 3H), 4.31 (br s, 3H), 3.81 (s, 3H), 1.64 - 1.59 (m, 3H).

### Step 5: Synthesis of Compound 001

**001-6** (0.02 g, 45.73 µmol, 1 *eq*) was dissolved in DMSO (2 mL). Then triethylamine (69.40 mg, 685.88 µmol, 95.47 µL, 15 *eq*)*,* HATU (156.47 mg, 411.53 µmol, 9 *eq*)*,* and ammonium chloride (36.69 mg, 685.88 µmol, 15 *eq*) were added thereto. The mixture was stirred at 25°C for 2 hours after the system was replaced with nitrogen. Three reactions were run in parallel. After the reaction was completed, the reaction mixtures were combined and quenched slowly with saturated sodium carbonate (50 mL), and then extracted with ethyl acetate (50 mL * 3). The organic phases were combined, washed with saturated sodium chloride (30 mL), then dried over anhydrous sodium sulfate, filtered, and finally evaporated to dryness by rotary evaporation under reduced pressure. The crude product was separated by preparative high performance liquid chromatography (chromatographic column: Phenomenex Gemini-NX 80 * 40 mm * 3 µm; mobile phase: [water (10 mMNH₄HCO₃)-ACN]; acetonitrile: 15% to 45%, 8 min) to obtain compound **001-7,** which was detected as a racemate. Compound **001-7** was separated by preparative supercritical fluid chromatography (chromatographic column: REGIS (s,s) WHELK-O1 (250 mm * 30 mm,5 µm); mobile phase: A was CO₂, B was [0.1% NH₃H₂O EtOH]; B%: 45% to 45%, 15 min), to obtain compounds **001** (Rt = 1.663 min) and **002** (Rt = 1.861 min).

Compound **001:** ¹H NMR (400 MHz, CD₃OD) δ 8.05 (d*, J* = 8.8 Hz, 1H), 7.16 (s, 1H), 6.81 - 6.39 (m, 2H), 6.18 (d, *J* = 2.4 Hz, 1H), 4.76 - 4.52 (m, 3H), 4.43 - 4.38 (m, 2H), 4.34 (br s, 2H), 3.82 (q, *J* = 7.0 Hz, 1H),3.31(s, 3H), 1.46 (d, *J* = 7.1 Hz, 3H); MS: m/z = 437 [M+1]⁺; ee% = 98.8%.

Compound **002:** ¹H NMR (400 MHz, CD₃OD) δ 8.04 (d, *J* = 8.8 Hz, 1H), 7.06 (s, 1H), 6.62 - 6.34 (m, 2H), 6.17 (d, *J* = 2.4 Hz, 1H), 5.01 - 4.91 (m, 1H), 4.71 (dd, *J* = 3.3, 9.3 Hz, 1H), 4.65 - 4.56 (m, 1H), 4.42 - 4.36 (m, 2H), 4.31 (dt, *J* = 1.8, 4.0 Hz, 2H), 3.84 - 3.79 (m, 1H), 3.68 (s, 3H), 1.46 (d, *J* = 7.1 Hz, 3H); MS: m/z = 437 [M+1] ⁺; ee% = 98.7%.

### Example 2

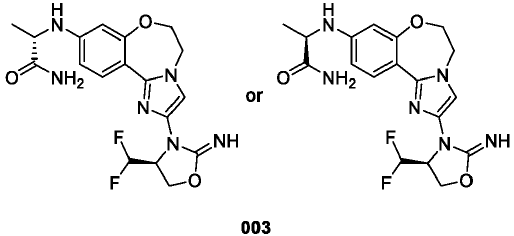

### Synthetic Route:

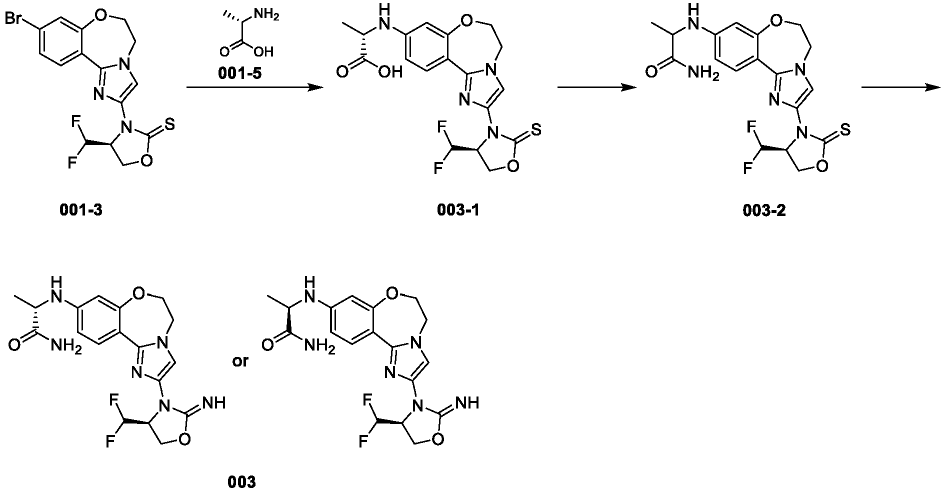

### Step 1: Synthesis of Compound 003-1

**001-3** (0.2 g, 480.49 µmol, 1 *eq*)*,* **001-5** (171.23 mg, 1.92 mmol, 4 *eq*)*,* and potassium phosphate (815.95 mg, 3.84 mmol, 8 *eq*) were dissolved in DMSO (10 mL). After the system was replaced with nitrogen, copper iodide (118.96 mg, 624.64 µmol, 1.3 *eq*) was added thereto, and the reaction mixture was heated to 90°C under microwave irradiation and stirred for 0.5 hours. Ten reactions were run in parallel. After the reaction was completed, 10 reaction mixtures were combined, and then poured into 200 mL of ice water in an ice-water bath for dilution, filtered, and then the filtrate was extracted with ethyl acetate (100 mL). After phase separation, the aqueous phase was collected, and the pH of the aqueous phase was adjusted to about 6 with 10% sodium bisulfate, and the aqueous phase was extracted with ethyl acetate (200 mL * 3). The organic phases were combined, washed with saturated brine (100 mL), dried over anhydrous sodium sulfate, and concentrated under reduced pressure to obtain compound **003-1,** which was directly used in the next reaction step.

### Step 2: Synthesis of Compound 003-2

**003-1** (1.2 g, 2.83 mmol, 1 *eq*) was dissolved in tetrahydrofuran (120 mL), then HOSu (1.95 g, 16.96 mmol, 6 *eq*) was added thereto. After the mixture was stirred at 25°C for 0.5 hours, EDCI (5.42 g, 28.27 mmol, 10 *eq*) and NH₃/MeOH (7 M, 6.06 mL, 15 *eq*) were added thereto in sequence. The mixture was stirred at 25°C for 9.5 hours after the system was replaced with nitrogen. After the reaction was completed, 100 mL of water/100 mL of ethyl acetate was added to the reaction system for dilution. The organic phase was collected after phase separation. The aqueous phase was extracted with ethyl acetate (50 mL * 3). The organic phases were combined, washed with saturated brine (100 mL), dried over anhydrous sodium sulfate, and concentrated under reduced pressure. The crude product was separated by silica gel column chromatography (silica gel mesh: 100 to 200 mesh; dichloromethane: methanol=100:0 to 100:3) to obtain compound **003-2.** ¹H NMR (400 MHz, CDCl₃) δ 8.17 - 8.10 (m, 1H), 8.00 (s, 1H), 6.85 - 6.45 (m, 2H), 6.30 - 6.20 (m, 1H), 5.28 - 5.16 (m, 1H), 4.93 (dd, *J* = 3.9, 9.7 Hz, 1H), 4.74 - 4.66 (m, 1H), 4.48 - 4.43 (m, 2H), 4.38 - 4.34 (m, 2H), 3.94 - 3.84 (m, 1H), 1.57 (d, *J* = 7.0 Hz, 3H).

### Step 3: Synthesis of compound 003

**003-2** (0.1 g, 236.16 µmol, 1 *eq*)*,* silver acetate (78.84 mg, 472.33 µmol, 24.18 µL, 2 *eq*)*,* and a solution (7 M, 4.00 mL, 118.56 *eq*) of ammonia in methanol were added to a dry reaction flask. The reaction mixture was stirred at 60°C for 2 hours. After the reaction was completed, the reaction mixture was filtered, and finally evaporated by rotary evaporation under reduced pressure. The crude product was separated by preparative high performance liquid chromatography (chromatographic column: Phenomenex luna C18 100 * 40 mm * 5 µm; mobile phase: [Water (0.1%TFA)-ACN]; acetonitrile: 1% to 20%, 8 min) to obtain compound **003.** ¹H NMR (400 MHz, CD₃OD) δ 8.08 (d, *J* = 8.8 Hz, 1H), 7.31 (s, 1H), 6.67 - 6.24 (m, 2H), 6.20 (d, *J* = 2.3 Hz, 1H), 5.14 - 4.95 (m, 3H), 4.51 - 4.34 (m, 4H), 3.84 (d, *J* = 7.0 Hz, 1H), 1.47 (d, *J* = 7.0 Hz, 3H); MS: m/z = 407 [M+1] ⁺; ee%=95.5% (SFC Rt = 1.142 min).

### Example 3

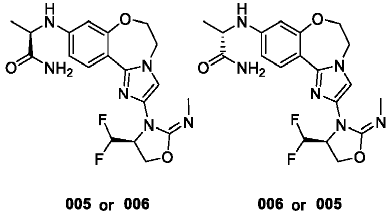

### Synthetic Route:

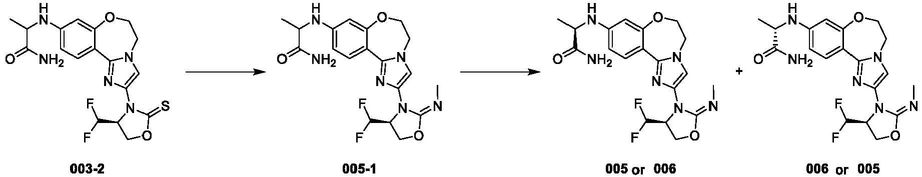

### Step 1: Synthesis of compound 006

**003-2** (0.2 g, 472.33 µmol, 1 *eq*) and silver acetate (157.67 mg, 944.65 µmol, 48.37 µL, 2 *eq*) were added to a dry reaction flask. Then DMF (5 mL) was added thereto. After the system was replaced with nitrogen, methylamine hydrochloride (63.78 mg, 944.65 µmol, 2 *eq*) and triethylamine (191.18 mg, 1.89 mmol, 262.97 µL, 4 *eq*) were added thereto. The reaction mixture was stirred at 25°C for 10 hours. After the reaction was completed, the reaction mixture was filtered, and finally evaporated to dryness by rotary evaporation under reduced pressure. The crude product was separated by preparative high performance liquid chromatography (chromatographic column: Phenomenex luna CN 5 µm 100 * 30 mm; mobile phase: [n-heptane-EtOH]; ethanol: 40% to 95%, 10 min) to obtain compound **005-1,** and then separated by preparative supercritical fluid chromatography (chromatographic column: REGIS (S, S) WHELK-O1 (250 mm * 25 mm, 10 µm); mobile phase: A was CO₂, B was [neutral-IPA]; B%: 50% to 50%, 15 min) to obtain compound **005** (Rt = 1.646 min) or **006** (Rt = 1.844 min).

Compound **006:** ¹H NMR (400 MHz, CD₃OD) δ 8.01 (d, *J* = 8.8 Hz, 1H), 7.10 (s, 1H), 6.57 - 6.22 (m, 2H), 6.18 (d, *J* = 2.1 Hz, 1H), 4.74 - 4.55 (m, 3H), 4.43 - 4.37 (m, 2H), 4.33 - 4.28 (m, 2H), 3.82 (q, *J* = 7.0 Hz, 1H), 2.88 (s, 3H), 1.46 (d, *J* = 6.9 Hz, 3H); LCMS m/z = 421 [M+1]⁺.

### Example 4

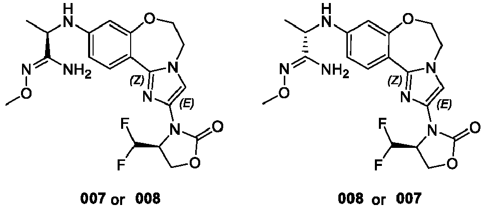

### Synthetic Route:

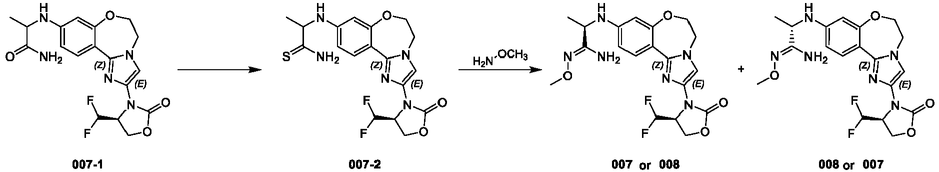

### Step 1: Synthesis of Compound 007-2

**007-1** (250 mg, 613.69 µmol, 1 eq) and Lawesson's reagent (744.66 mg, 1.84 mmol, 3 eq) were added to a pre-dried reaction flask, followed by the addition of solvent tetrahydrofuran (1 mL). The reaction was stirred at 20°C for 1 hour. After the reaction was completed, the reaction mixture was directly evaporated to dryness by rotary evaporation to obtain the crude product. The crude product was separated by silica gel column chromatography (silica gel mesh: 100 to 200 mesh; eluted with petroleum ether: ethyl acetate = 20:1 for 30 min, and the eluent was switched to dichloromethane: methanol = 1:0 to 50:1) to obtain compound **007-2.** ¹H NMR (400 MHz, CDCl₃) δ 8.18 (d, *J*=8.77 Hz, 1H), 8.10 (br s, 1H), 7.43 (br s, 1H), 7.18 (s, 1H), 6.48-6.79 (m, 1H), 6.44 (dd, *J*=2.41, 8.77 Hz, 1H), 6.23 (d, *J*=2.41 Hz, 1H), 4.94 (br d, *J*=13.59 Hz, 1H), 4.71 (dd, *J*=3.84, 9.32 Hz, 1H), 4.51-4.59 (m, 1H), 4.42 (br d, *J*=7.02 Hz, 2H), 4.23-4.33 (m, 4H), 1.68 (s, 3H).

### Step 2: Synthesis of Compounds 007 and 008

**007-2** (30.00 mg, 70.85 µmol, 1 *eq*) and solvent dichloromethane (2 mL) were added to the pre-dried reaction flask. The mixture was cooled to 0°C, added with methyl trifluoromethanesulfonate (69.76 mg, 425.09 µmol, 46.51 µL, 6 *eq*)*,* heated to 20°C, and stirred for 2 hours. The reaction system was cooled to 0°C, and O-methylhydroxylamine hydrochloride (16.67 mg, 199.59 µmol, 3.97 µL, 2.82 *eq*) and DIEA (54.94 mg, 425.09 µmol, 74.04 µL, 6 *eq*) were added thereto. The reaction was stirred at 20°C for 10 hours. After the reaction was completed, the reaction mixture was evaporated to dryness by rotary evaporation, and the crude product was separated by high performance liquid chromatography (chromatographic column: Phenomenex Gemini-NX 150 * 30 mm * 5 µm; mobile phase: [Water (0.1% TFA)-ACN]; acetonitrile: 5% to 35%, 9 min), and then separated by preparative supercritical fluid chromatography (chromatographic column: DAICEL CHIRALCEL OJ (250 mm*30 mm, 10 µm); mobile phase: A was CO₂, B was [0.1% NH₃H₂OMeOH]; B%: 45% to 45%, 10 min) to obtain compound **007** (Rt = 1.231 min) and compound **008** (Rt = 1.428 min).

Compound **007:** ¹H NMR (400 MHz, CDCl₃) δ 8.14 (d, *J*=8.78 Hz, 1H), 7.17 (s, 1H), 6.53-6.84 (m, 1H), 6.48 (dd, *J*=2.32, 8.85 Hz, 1H), 6.34 (d, *J*=2.26 Hz, 1H), 4.82-4.93 (m, 1H), 4.72 (dd, *J*=3.95, 9.35 Hz, 1H), 4.67 (s, 2H), 4.49-4.55 (m, 1H), 4.42 (br d, *J*=4.89 Hz, 2H), 4.30 (br d, *J*=5.40 Hz, 2H), 3.98 (br dd, *J*=3.14, 6.65 Hz, 1H), 3.92 (br s, 1H), 3.84 (s, 3H), 1.54 (d, *J*=6.78 Hz, 3H); MS: m/z = 437.2 [M+1] ⁺; ee% = 99.24%.

Compound **008:** ¹H NMR (400 MHz, CDCl₃) δ 8.13 (d, *J*=8.78 Hz, 1H), 7.16 (s, 1H), 6.53-6.87 (m, 1H), 6.48 (dd, *J*=2.20, 8.72 Hz, 1H), 6.34 (d, *J*=2.26 Hz, 1H), 4.79-4.95 (m, 1H), 4.72 (dd, *J*=4.02, 9.41 Hz, 1H), 4.68 (s, 2H), 4.48-4.56 (m, 1H), 4.38-4.45 (m, 2H), 4.26-4.32 (m, 2H), 3.95-4.04 (m, 1H), 3.93 (br d, *J*=3.64 Hz, 1H), 3.83 (s, 3H), 1.53 (d, *J*=6.65 Hz, 3H); MS: m/z = 437.2 [M+1] ⁺; ee%=100%.

The detection and analysis method of supercritical fluid chromatography for ee% value of above compounds **007** and **008** was as follows: (chromatographic column: DAICEL CHIRALCEL OJ (150 mm * 4.6 mm, 5 µm); mobile phase: A was CO₂, B was [0.05% DEA EtOH]; B%: 5% to 40%, 10 min).

### Example 5

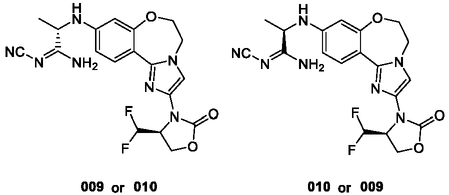

### Synthetic Route:

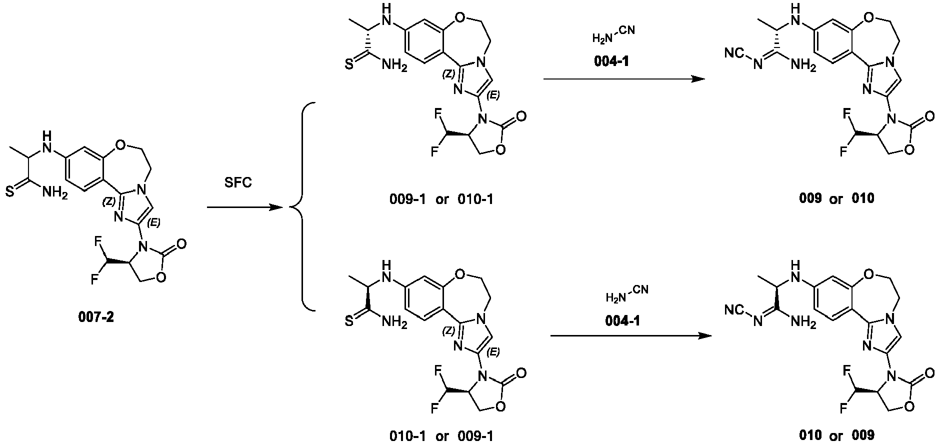

### Step 1: Synthesis of Compounds 009-1 and 010-1

**007-2** (100 mg, 236.16 µmol, 1 *eq*) was separated by preparative supercritical fluid chromatography (chromatographic column: DAICEL CHIRALPAK AS (250 mm * 30 mm, 10 µm); mobile phase: A was CO₂, B was [0.1% NH₃H₂O EtOH]; B%: 50% to 50%, 8 min) to obtain compounds **009-1** (RT = 1.425 min) and **010-1** (RT = 1.584 min).

### Step 2: Synthesis of Compound 009

**009-1** (50 mg, 118.08 µmol, 1 *eq*) and dichloromethane (2 mL) were added to a pre-dried reaction flask. The mixture was cooled to 0°C, added with methyl trifluoromethanesulfonate (38.75 mg, 236.16 µmol, 25.84 µL, 2 *eq*)*,* heated to 20°C, and stirred for 2 hours. The reaction system was cooled to 0°C. **004-1** (24.82 mg, 590.41 µmol, 24.82 µL, 5 *eq*) and DIEA (30.52 mg, 236.16 µmol, 41.13 µL, 2 *eq*) were added thereto. The reaction was stirred at 20°C for 10 hours. After the reaction was completed, the reaction mixture was directly evaporated to dryness by rotary evaporation, and was separated by preparative high performance liquid chromatography (chromatographic column: Phenomenex Gemini-NX 150 * 30 mm * 5 µm; mobile phase: [Water (0.1% TFA)-ACN]; acetonitrile: 12 % to 27 %, 9 min) to obtain the trifluoroacetate of compound **009.** ¹H NMR (400 MHz, CD₃OD) δ 8.54 (d, *J*=8.50 Hz, 1H), 7.37 (s, 1H), 7.10-7.21 (m, 2H), 6.49-6.85 (m, 1H), 5.19 (q, *J*=7.00 Hz, 1H), 4.94-5.05 (m, 1H), 4.68-4.75 (m, 1H), 4.61-4.67 (m, 1H), 4.53-4.58 (m, 2H), 4.46-4.52 (m, 2H), 1.48 (d, *J*=7.00 Hz, 3H). MS (1.5 min); m/z = 432.2 [M+1] ⁺; SFC Rt=1.254 min; ee%=100%.

### Step 3: Synthesis of Compound 010

**010-1** (20.00 mg, 47.23 µmol, 1 *eq*) and solvent dichloromethane (2 mL) were added to the pre-dried reaction flask. The mixture was cooled to 0°C, added with methyl trifluoromethanesulfonate (15.50 mg, 94.47 µmol, 10.33 µL, 2 *eq*)*,* heated to 20°C, and stirred for 2 hours. The reaction system was cooled to 0°C. **004-1** (9.93 mg, 236.16 µmol, 9.93 µL, *5 eq*) and DIEA (12.21 mg, 94.47 µmol, 16.45 µL, 2 *eq)* were added thereto. The reaction was stirred at 20°C for 10 hours. After the reaction was completed, the reaction mixture was directly evaporated to dryness by rotary evaporation. The crude product was separated by preparative high performance liquid chromatography (chromatographic column: Phenomenex Gemini-NX 150 * 30 mm * 5 µm; mobile phase: [Water (0.1% TFA)-ACN]; acetonitrile: 12 % to 27 %, 9 min) to obtain the trifluoroacetate of compound **010.** ¹H NMR (400 MHz, CD₃OD) δ 8.52 (d, *J*=8.60 Hz, 1H), 7.35 (s, 1H), 7.06-7.18 (m, 2H), 6.48-6.81 (m, 1H), 5.15 (q, *J*=6.91 Hz, 1H), 4.99 (br d, *J*=9.26 Hz, 1H), 4.66-4.70 (m, 1H), 4.59-4.64 (m, 1H), 4.51-4.57 (m, 2H), 4.45-4.49 (m, 2H), 1.45 (d, *J*=7.06 Hz, 3H); m/z = 432.0 [M+1] ⁺; SFC Rt=1.197 min.

### Example 6

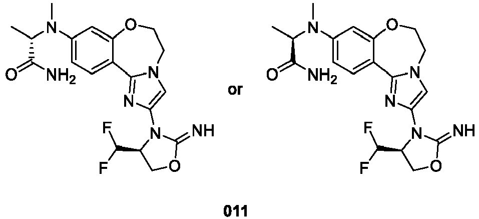

### Synthetic Route:

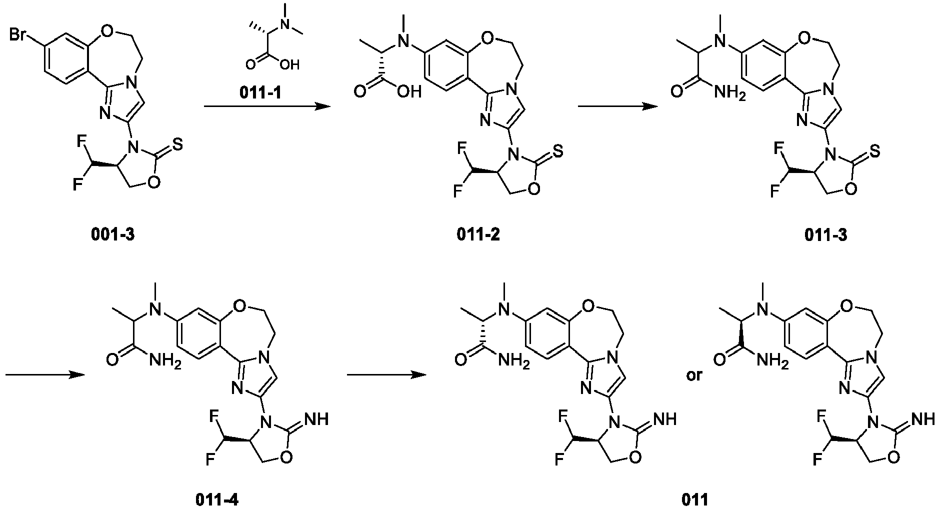

### Step 1: Synthesis of Compound 011-2

Three 20 mL microwave tubes were taken, and three reactions were run in parallel as follows. **001-3** (0.2 g, 480.49 µmol, 1 *eq*)*,* **011-1** (198.19 mg, 1.92 mmol, 4 *eq*)*,* and potassium phosphate (815.95 mg, 3.84 mmol, 8 *eq*) were dissolved in DMSO (10 mL). After the system was replaced with nitrogen, copper iodide (118.96 mg, 624.64 µmol, 1.3 *eq*) was added thereto, and the reaction mixture was heated to 90°C under microwave irradiation and stirred for 80 minutes. After the reaction was completed, the three reactions were combined for processing. The reaction mixture was poured into 20 mL of ice water in an ice-water bath for dilution and filtered. The filtrate was extracted with ethyl acetate (100 mL). After phase separation, the aqueous phase was collected, and the pH of the aqueous phase was adjusted to about 6 with 10% sodium bisulfate, and the aqueous phase was extracted with ethyl acetate (200 mL * 3). The organic phases were combined, washed with saturated brine (100 mL), dried over anhydrous sodium sulfate, filtered and concentrated under reduced pressure to obtain compound **011-2,** which was directly used in the next reaction step.

### Step 2: Synthesis of Compound 011-3

**011-2** (0.6 g, 1.37 mmol, 1 *eq*) was dissolved in tetrahydrofuran (10 mL), and then HOSu (944.96 mg, 8.21 mmol, 6 *eq*) was added thereto. After stirring at 25°C for 0.5 hours, EDCI (2.62 g, 13.68 mmol, 10 *eq*) and a solution (7 M, 2.93 mL, 15 *eq*) of ammonia in methanol were thereto added in sequence. After the system was replaced with nitrogen, the mixture was stirred at 25°C for 9.5 hours. After the reaction was completed, the reaction mixture was quenched by adding water (5 mL), then extracted with ethyl acetate (100 mL * 2). The organic phases were collected, combined and evaporated to dryness by rotary evaporation under reduced pressure. The crude product was separated by preparative high performance liquid chromatography (chromatographic column: Phenomenex Gemini-NX 80 * 40 mm * 3 µm; mobile phase: [Water (0.05% NH₃H₂O)-ACN]; acetonitrile: 32% to 62%, 8 min) to obtain compound **011-3.** ¹H NMR (400 MHz, CD₃OD) δ ppm 1.41 (d, *J*=7.03 Hz, 3 H), 2.91 (s, 3 H), 4.35 - 4.40 (m, 2 H), 4.41 - 4.45 (m, 2 H), 4.49 (q, *J*=7.03 Hz, 1 H), 4.69 - 4.79 (m, 1 H), 4.85 (d, *J*=3.76 Hz, 1 H), 5.21 - 5.35 (m, 1 H), 6.42 (d, *J*=2.51 Hz, 1 H), 6.45 - 6.78 (m, 2 H), 7.89 (s, 1 H), 8.15 (d, *J*=9.03 Hz, 1 H).

### Step 3: Synthesis of Compound 011

To a dry reaction flask was added **011-3** (0.2 g, 457.18 µmol, 1 *eq*)*,* silver acetate (152.62 mg, 914.36 µmol, 46.82 µL, 2 *eq*)*,* and a solution (10 mL) of ammonia in methanol. The mixture was heated to 60°C and reacted for 2 hours. After the reaction was completed, the reaction mixture was filtered, and the filtrate was collected. The filter cake was rinsed with methanol (10 mL). The organic phases were combined and evaporated to dryness by rotary evaporation. The crude product was separated by preparative high performance liquid chromatography (chromatographic column: Phenomenex Gemini-NX C18 75 * 30 mm * 3 µm; mobile phase: [Water (0.225% FA)-ACN]; acetonitrile: 5% to 35%, 7 min) to obtain compound **011-4,** and then separated by preparative supercritical fluid chromatography (chromatographic column: DAICEL CHIRALCEL OJ (250 mm * 30 mm, 10 µm); mobile phase: A was CO₂, B was [0.1% NH₃H₂O EtOH]; B%: 35% to 35%, 8 min) to obtain compound **011** (Rt = 3.405 min). ¹H NMR (400 MHz, CD₃OD) δ ppm 1.40 (d, *J*=7.03 Hz, 3 H), 2.91 (s, 3 H), 4.32 - 4.39 (m, 2 H), 4.40 - 4.45 (m, 2 H), 4.48 (q, *J*=7.03 Hz, 1 H), 4.52 - 4.62 (m, 2 H), 4.67 - 4.79 (m, 1 H), 6.19 - 6.52 (m, 2H), 6.65 (dd, *J*=9.03, 2.51 Hz, 1 H), 7.06 - 7.20 (m, 1 H), 8.11 (d, *J*=9.03 Hz, 1 H); MS: m/z = 421.0 [M+1] ⁺; ee% = 100%.

### Example 7

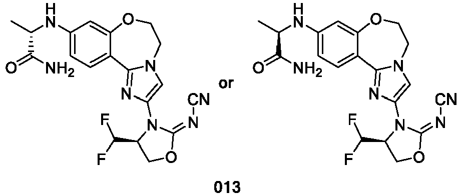

### Synthetic Route:

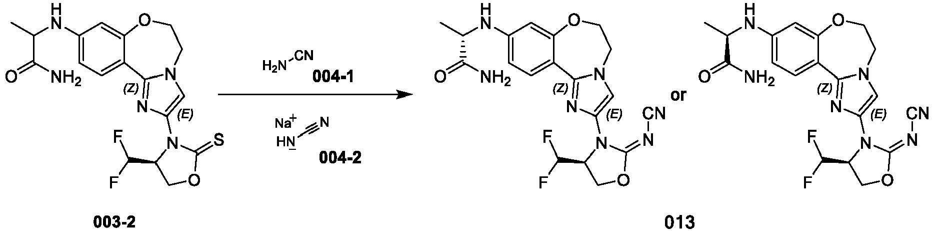

### Step 1: Synthesis of Compound 013

**003-2** (50 mg, 118.08 µmol, 1 *eq*) and solvent DMF (3 mL) were added to a pre-dried reaction flask. Then **004-2** (15.36 mg, 236.16 µmol, 9.93e-1 µL, 2 *eq*)*,* **004-1** (9.93 mg, 236.16 µmol, 9.93 µL, 2 *eq*), and silver acetate (39.42 mg, 236.16 µmol, 12.09 µL, 2 *eq*) were added thereto. The reaction was stirred at 20°C for 1 hour. After the reaction was completed, the reaction mixture was filtered, and the filtrate was diluted with 10 mL of water/10 mL of ethyl acetate. After phase separation, the organic phase was collected, and the aqueous phase was extracted with ethyl acetate (5 mL * 3). The organic phases were combined, washed with saturated brine (20 mL), dried over anhydrous sodium sulfate, filtered, and concentrated under reduced pressure to obtain a crude product. The crude product was separated by preparative high performance liquid chromatography (chromatographic column: Waters Xbridge BEH C18 100 * 30 mm * 10 µm; mobile phase: [water (10mM NH₄HCO₃)-ACN]; acetonitrile: 10% to 40%, 8 min), and then separated by preparative supercritical fluid chromatography (chromatographic column: DAICEL CHIRALCEL OD (250 mm * 30 mm, 10 µm); mobile phase: A was CO₂, B was neutral MeOH]; B%: 50% to 50%, 10 min) to obtain compound **013** (Rt = 1.598 min). 1H NMR (400 MHz, DMSO-*d*₆) δ 7.94 (d, *J*=8.82 Hz, 1H), 7.33 (s, 1H),7.20 (s, 1H), 6.95 (s, 1H), 6.51-6.84 (m, 1H), 6.35 (dd, *J*=2.43, 8.82 Hz, 1H), 6.15 (d, *J*=7.06 Hz, 1H), 6.03 (d, *J*=2.43 Hz, 1H), 5.12-5.25 (m, 1H), 4.83-4.90 (m, 2H), 4.30 (s, 4H), 3.71 (quin, *J*=6.84 Hz, 1H), 1.25 (d, *J*=6.84 Hz, 3H); MS : m/z = 432.1 [M+1] ⁺.

### Example 8

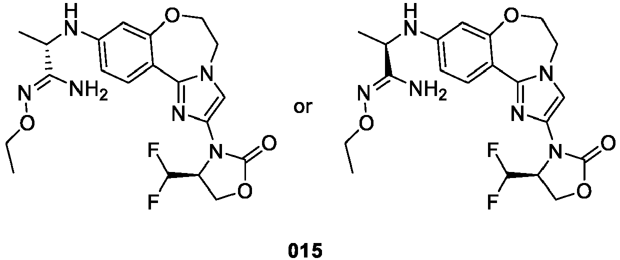

### Synthetic Route:

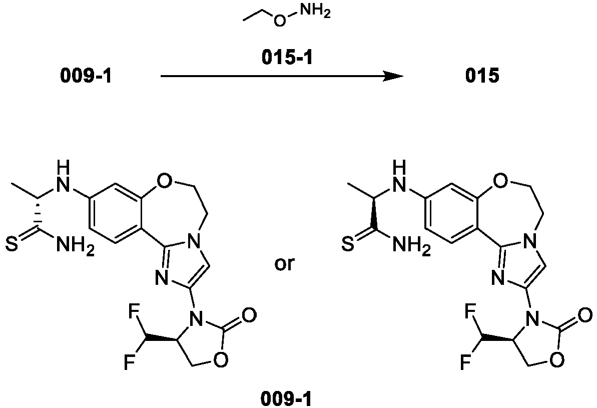

### Step 1: Synthesis of Compound 015

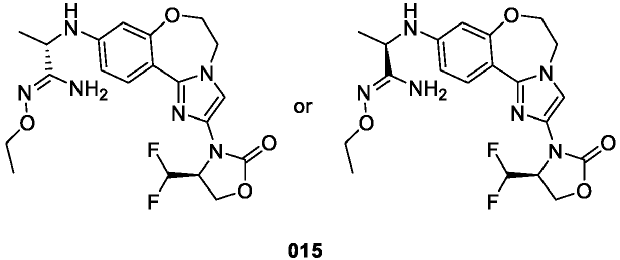

Compound **009-1** (30.00 mg, 70.85 µmol, 1 eq) was added to a pre-dried reaction flask. Solvent dichloromethane (3.5 mL) was added thereto. The mixture was cooled to 0°C, and added with methyl trifluoromethanesulfonate (58.13 mg, 354.25 µmol, 38.75 µL, 5 eq). The mixture was heated to 20°C and stirred for 2 hours. The reaction system was cooled to 0°C, and **015-1** (HCl, 14.89 µL, 5 eq) and DIEA (45.78 mg, 354.25 µmol, 61.70 µL, 5 eq) were added thereto. The reaction was stirred at 20°C for 10 hours. After the reaction was completed, the reaction mixture was quenched with methanol and evaporated to dryness by rotary evaporation to obtain a crude product. The crude product was separated by preparative high performance liquid chromatography (chromatographic column: Phenomenex Luna C18 150 * 30 mm * 5 µm; mobile phase: [Water (0.1% TFA)-ACN]; B (acetonitrile)%: 1% to 35%, 9 min), then resolved by preparative supercritical fluid chromatography (chromatographic column: DAICEL CHIRALPAK IC (250 mm * 30 mm, 10 µm); mobile phase: [0.1% NH₃H₂O MeOH]; B%: 50% to 50%, 10 min) to obtain compound **015** (Rt = 1.375 min). ¹H NMR (400 MHz, CD₃OD) δ ppm 1.22 - 1.30 (m, 3 H) 1.49 (d, *J*=6.88 Hz, 3 H) 3.83 - 3.93 (m, 1 H) 3.94 - 4.04 (m, 2 H) 4.30 - 4.36 (m, 2 H) 4.38 - 4.46 (m, 2 H) 4.59 - 4.64 (m, 1 H) 4.66 - 4.73 (m, 1 H) 4.93 - 5.01 (m, 1 H) 5.47 - 5.48 (m, 1 H) 6.34 (d, *J*=2.38 Hz, 1 H) 6.43 - 6.79 (m, 2 H) 7.17 (s, 1 H) 8.04 (d, *J*=8.75 Hz, 1 H); MS: m/z = 451.1 [M+1] ⁺.

### Example 9

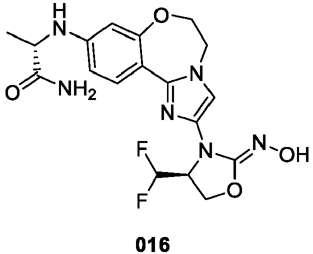

### Old Synthetic Route:

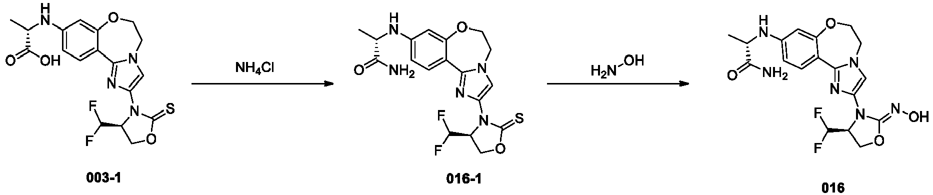

### Step 1: Synthesis of Compound 016-1

Compound **003-1** (0.13 g, 306.30 µmol, 1 eq) was dissolved in tetrahydrofuran (5 mL). HATU (1.75 g, 4.59 mmol, 15 eq), triethylamine (619.88 mg, 6.13 mmol, 852.66 µL, 20 eq), and ammonium chloride (245.77 mg, 4.59 mmol, 15 eq) were added at 0°C, and the mixture was stirred at 15°C for 16 hours. After the reaction was completed, water (20 mL) was added to the reaction mixture. The reaction mixture was extracted three times with ethyl acetate (3 × 10 mL). The organic phases were combined, dried over anhydrous sodium sulfate, filtered, and concentrated to obtain a crude product. The crude product was separated by preparative thin-layer chromatography (dichloromethane: methanol = 20:1) to obtain **016-1.** MS: m/z = 424 [M+1]⁺.

### Step 2: Synthesis of Compound 016

Compound **016-1** (130.00 mg, 307.01 µmol, 1 eq) was dissolved in methanol (10 mL). Triethylamine (155.33 mg, 1.54 mmol, 213.66 µL, 5 eq) and hydroxylamine hydrochloride (50.70 mg, 1.54 mmol, 5 eq) were added at 20°C. The mixture was heated to 80°C and reacted for 22 hours. After the reaction was completed, water (20 mL) was added to the reaction mixture. The reaction mixture was extracted three times with dichloromethane (3 × 10 mL). The organic phases were combined, dried over anhydrous sodium sulfate, filtered, and concentrated. Then the crude product was separated by preparative high performance liquid chromatography (chromatographic column: Boston Prime C18 150 * 30 mm * 5 µm; mobile phase: [Water (NH₃H₂O + NH₄HCO₃)-ACN]; 15% to 45%, 7 min) to obtain compound **016.** ¹H NMR (400MHz, CD₃OD) δ = 8.06 (d, *J*=9.0 Hz, 1H), 6.65 - 6.31 (m, 2H), 6.19 (d, *J*=2.3 Hz, 1H), 4.73 (dd, *J*=3.1, 9.2 Hz, 1H), 4.67 - 4.57 (m, 2H), 4.40 (br d, *J*=2.8 Hz, 2H), 4.32 (br d, *J*=3.3 Hz, 2H), 3.84 (q, *J*=6.8 Hz, 1H), 1.48 (d, *J*=7.0 Hz, 3H); MS: m/z = 423 [M+1]⁺.

### Example 10

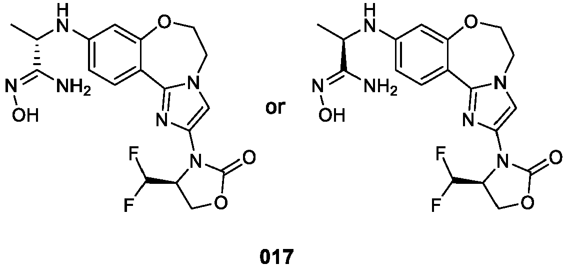

### Synthetic Route:

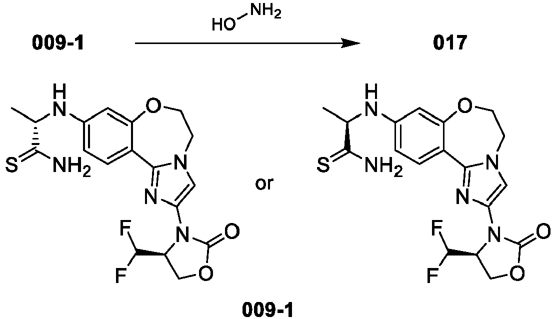

### Step 1: Synthesis of Compound 017

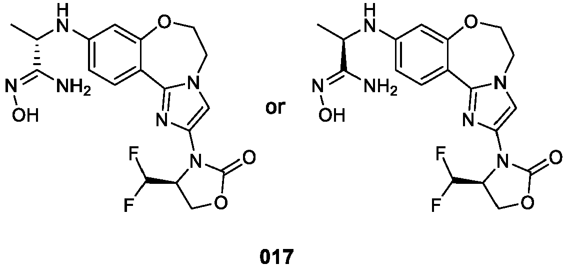

Under nitrogen atmosphere, hydroxylamine hydrochloride (66 mg, 949.76 µmol, 5.03 *eq*) was added to a reaction flask containing compound **009-1** (80 mg, 188.93 µmol, 1 *eq*)*,* TEA (98.15 mg, 969.92 µmol, 135 µL, 5.13 *eq*)*,* and methanol (5 mL). The mixture was heated to 80°C and stirred for 10 hours. After the reaction was completed, the reaction mixture was evaporated to dryness by rotary evaporation under reduced pressure, added with 20 mL of water, extracted three times with dichloromethane (20 mL × 3). The organic phase was collected, washed with saturated brine (20 mL), and dried over anhydrous sodium sulfate. The reaction mixture was separated by preparative thin-layer chromatography (dichloromethane: methanol=20:1), then resolved by preparative supercritical fluid chromatography (chromatographic column: DAICEL CHIRALPAK IC (250 mm * 30 mm, 10 µm); mobile phase: [0.1% NH₃H₂O MEOH]; B%: 50% to 50%, 10 min) to obtain compound **017.** ¹H NMR (400 MHz, CDCl₃) δ ppm 1.53 (br d, *J*=6.53 Hz, 3 H) 3.87 - 4.06 (m, 2 H) 4.29 (br d, *J*=4.52 Hz, 2 H) 4.40 (br s, 2 H) 4.47 - 4.60 (m, 1 H) 4.68 - 4.77 (m, 3 H) 4.79 - 4.92 (m, 1 H) 6.33 (s, 1 H) 6.47 (br d, *J*=8.78 Hz, 1 H) 6.52 - 6.87 (m, 1 H) 7.17 (s, 1 H) 8.13 (d, *J*=8.78 Hz, 1 H). MS: m/z = 423 [M+1]⁺.

### Example 11

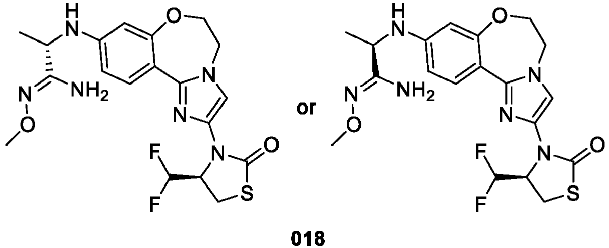

### Synthetic Route:

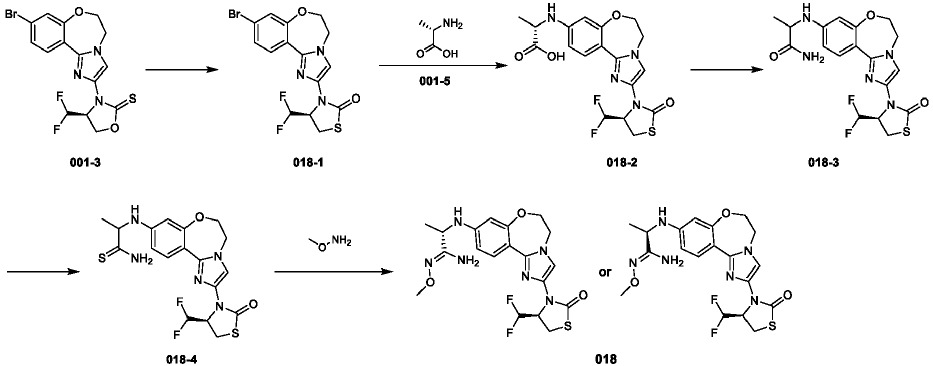

### Step 1: Synthesis of Compound 018-1

Compound **001-3** (0.87 g, 1.88 mmol, 1 eq) was dissolved in toluene (10 mL). Dichloro(p-cymene)ruthenium(II) dimer (345.94 mg, 564.90 µmol, 3.01 e-1 eq) and 2-dicyclohexylphosphino-2',6'-dimethoxybiphenyl (233.62 mg, 569.07 µmol, 3.03e-1 eq) were added. The mixture was heated to 110°C under nitrogen atmosphere and stirred for 12 hours. After the reaction was completed, the reaction mixture was cooled to room temperature (25°C), added with 10 mL of ethyl acetate and 10 mL of saturated brine. The organic phase was collected, dried over anhydrous sodium sulfate, evaporated to dryness by rotary evaporation under reduced pressure, and then separated by column chromatography (petroleum ether: ethyl acetate = 1:0 to 21:4) to obtain compound **018-1.** ¹H NMR (400 MHz, CDCl₃) δ ppm 3.59 (br d, *J*=11.80 Hz, 1 H) 3.69 - 3.80 (m, 1 H) 4.33 - 4.54 (m, 6 H) 5.18 - 5.34 (m, 1 H) 6.26 - 6.63 (m, 1 H) 7.38 - 7.50 (m, 3 H) 8.06 - 8.15 (m, 1 H).

### Step 2: Synthesis of Compound 018-2

Compound **018-1** (670 mg, 1.45 mmol, 1 *eq*)*,* **001-5** (520 mg, 5.84 mmol, 4.04 *eq*)*,* and potassium phosphate (1.58 g, 7.46 mmol, 5.16 *eq*) were dissolved in DMSO (20 mL). Copper iodide (365.45 mg, 1.92 mmol, 1.33 eq) was added thereto under nitrogen atmosphere. The mixture was heated to 125°C and stirred for 2 hours. After the reaction was completed, the reaction mixture was filtered. The filter cake was washed with 10 mL of DMSO. The filtrate was collected to obtain a DMSO solution of crude compound **018-2.** The reaction was directly used in the next step without further purification.

### Step 3: Synthesis of Compound 018-3

Compound **018-2** (600 mg, 1.41 mmol, 1 eq) was added to a pre-dried reaction flask. Then solvent DMSO (30 mL) and tetrahydrofuran (15 mL) were added thereto. The mixture was cooled to 0°C, added with HATU (3.25 g, 8.55 mmol, 6.05 eq), and then added with a solution (7 M, 4.5 mL, 22.28 eq) of ammonia in methanol. The reaction was stirred at 20°C for 12 hours. After the reaction was completed, the reaction mixture was distilled under reduced pressure. The reaction mixture was added with 100 mL of water, extracted three times with dichloromethane (50 mL * 3). The organic phase was collected, washed four times with water (100 mL * 4), and dried over anhydrous sodium sulfate. The organic phase was then separated by column chromatography (dichloromethane: methanol=1:0 to 9:1) to obtain compound **018-3.** Racemization was speculated to occur during the reaction. ¹H NMR (400 MHz, CD₃OD) δ ppm 2.12 (d, *J*=7.03 Hz, 3 H) 4.22 (dd, *J*=12.05, 2.26 Hz, 1 H) 4.38 - 4.51 (m, 2 H) 4.92 - 5.08 (m, 4 H) 5.24 (s, 3 H) 5.73 - 5.86 (m, 1 H) 6.82 (d, *J*=2.26 Hz, 1 H) 6.94 - 7.27 (m, 2 H) 7.92 (s, 1 H) 8.69 (d, *J*=9.03 Hz, 1 H).

### Step 4: Synthesis of Compound 018-4

Compound **018-3** (100 mg, 236.16 µmol, 1 eq) and Lawesson's reagent (190 mg, 469.75 µmol, 1.99 eq) were added to a pre-dried reaction flask, followed by solvent tetrahydrofuran (4 mL). The reaction was stirred at 20°C for 2 hours. After the reaction was completed, the reaction mixture was evaporated to dryness by rotary evaporation under reduced pressure. The crude product was separated by preparative thin-layer chromatography (dichloromethane: methanol = 20:1) to obtain compound **018-4.** ¹H NMR (400 MHz, CDCl₃) δ ppm 1.67 (br d, *J*=6.53 Hz, 3 H) 3.06 (q, *J*=7.19 Hz, 1 H) 3.54 - 3.73 (m, 2 H) 4.26 - 4.31 (m, 3 H) 4.39 - 4.46 (m, 2 H) 5.06 - 5.23 (m, 1 H) 6.23 (d, *J*=2.51 Hz, 1 H) 6.34 - 6.66 (m, 2 H) 7.32 (s, 1 H) 7.44 (br s, 1 H) 8.11 (br s, 1 H) 8.17 (d, *J*=8.53 Hz, 1 H).

### Step 5: Synthesis of Compound 018

Compound **018-4** (60 mg, 136.52 µmol, 1 eq) was added to the pre-dried reaction flask. Dichloromethane (3 mL) was added thereto, and the mixture was cooled to 0°C. Methyl trifluoromethanesulfonate (50.58 mg, 308.22 µmol, 33.72 µL, 2.26 eq) was added thereto, and the mixture was heated to 20°C and stirred for 2 hours. The reaction system was cooled to 0°C. O-methylhydroxylamine hydrochloride (120.40 mg, 1.44 mmol, 109.46 µL, 10.56 eq) and DIEA (221.61 mg, 1.71 mmol, 298.66 µL, 12.56 eq) were added thereto. The reaction was continued to be stirred at 20°C for 10 hours. After the reaction was completed, 50 mL of water was added to the reaction system. The organic phase was collected, and the aqueous phase was extracted with dichloromethane (50 mL * 3). The organic phases were combined, washed with saturated brine (50 mL), dried over anhydrous sodium sulfate, poured, concentrated under reduced pressure, separated by preparative thin-layer chromatography (dichloromethane: methanol = 20:1), and then resolved by preparative supercritical fluid chromatography (chromatographic column: DAICEL CHIRALPAK AS (250 mm * 30 mm, 10 µm); mobile phase: [0.1% NH₃H₂O EtOH]; B%: CO₂, 35% to 35%, 10 min) to obtain compound **018** (Rt = 2.041 min). ¹H NMR (400 MHz, CDCl₃) δ ppm 1.53 (d, *J*=6.78 Hz, 3 H) 3.58 - 3.71 (m, 2 H) 3.83 (s, 3 H) 3.90 - 4.01 (m, 2 H) 4.30 (br d, *J*=4.27 Hz, 2 H) 4.38 - 4.45 (m, 2 H) 4.68 (s, 2 H) 5.10 - 5.21 (m, 1 H) 6.33 (d, *J*=2.26 Hz, 1 H) 6.37 - 6.66 (m, 2 H) 7.31 (s, 1 H) 8.14 (d, *J*=8.78 Hz, 1 H); MS: m/z = 453.1 [M+1] ⁺.

### Experimental Example 1: In Vitro Evaluation

### 1. Enzyme activity test in vitro

The lipid kinase reaction was carried out under the conditions of the appropriate substrate and ATP. Then the kinase activity was tested with the ADP-Glo^{™} kit in two steps. Step 1: The kinase reaction was terminated, in which residual ATP was completely removed, and only ADP was remained; Step 2: Kinase test reagent was added to convert ADP into ATP, accompanied by luciferin/luciferase reaction. Finally, the fluorescence numerical output value was converted into kinase activity. The conditions for testing PI3K enzyme activity are shown in Table 1.

**Table 1 Conditions for testing PI3K enzyme activity**

| Subtype | Enzyme Final Concentration | ATP (µM) | PIP2:3PS (µM) | Reaction time (min) |
|---|---|---|---|---|
| PI3K α | 0.2 nM | 40 | 50 | 120 |
| PI3K β | 0.6 nM | 40 | 50 | 120 |
| PI3K δ | 0.25 nM | 40 | 50 | 120 |
| PI3K γ | 0.4 nM | 25 | 50 | 120 |

### Experimental materials and equipment:

a) Enzyme:

| | |
|---|---|
| PI3K α | Millipore #14-602-K |
| PI3K β | Promega #V1751 |
| PI3K δ | Millipore #14-604-K |
| PI3K γ | Millipore #14-558-K |

b) Kit: ADP-Glo^{™} Lipid Kinase and PIP2:3PS Kit (Promega #V1792)
The kit contains: 1 mM PIP2:3PS, 10 × lipid dilution buffer, 1 M magnesium chloride, 10 mM ATP, 10 mM ADP, ADP-Glo reagent, assay buffer, and assay substrate.
c) Reaction well plate: OptiPlate-384, white clear (PerkinElmer #6007299)

### Reagent preparation:

a) 10 × reaction buffer: 500 mM HEPES, pH 7.5, 500 mM NaCl, 9 mM MgCl₂; BSA: 10% stock solution, homemade
b) Final test system conditions: 1 × reaction system: 50 mM HEPES, 50 mM NaCl, 3 mM MgCl₂, 0.01% BSA (freshly prepared on the day of the experiment), 1% DMSO (v/v) + / - compound
c) Reaction system: 3 µL mixture of enzyme and substrate (1:1) + 2 µL ATP/MgCl₂ mixture + 5 µL ADP-Glo reagent + 10 µL assay reagent.

The specific experimental operations are as follows:
a) Compound dilution: 50 nL of 100× compound/DMSO was transferred to the test well plate with Echo
   - For PI3K α, the compounds were diluted three-fold from the highest concentration of 0.111 mM for a total of 10 concentrations.
   - For PI3K β/PI3K δ/PI3K γ, the compounds were diluted three-fold from the highest concentration of 1.11 mM for a total of 10 concentrations.
b) Kinase reaction:
   (1) The compound to be tested was prepared. 50 nL of 100 plus compound solution or DMSO was added to the corresponding well plate.
   (2) 3.33× reaction buffer was prepared.
   (3) 3.33× PIP2:3PS was prepared. PIP2:3PS was thawed by vortexing for at least 1 minute before use.
   (4) ATP solution containing 5.25 mM MgCl₂ was prepared.
   (5) 3.33× PI3K α/PI3K β/PI3K δ/PI3K γ solution was prepared.
   (6) The lipid kinase solution and the PIP2:3PS solution were mixed in a volume ratio of 1:1.
   (7) 3.33× lipid kinase buffer and PIP2:3PS solution were mixed in a volume ratio of 1:1.
   (8) 3 µL of the mixed solution of buffer and PIP2:3PS was added to column 1 and column 2 of the well plate.
   (9) 3 µL of the mixed solution of enzyme and PIP2:3PS was added to the wells of the well plate except the column 1 and column 2, and the plate was centrifuged for 10 seconds (1000 rpm). The plate was incubated at 23°C for 20 min.
   (10) 2 µL of ATP solution was added thereto, shaken uniformly at 1000 rpm.
   (11) The well plate was covered and shaken uniformly for about 30 seconds. Then the well plate was incubated at 23°C for 2 hours.
   (12) 5 µL of ADP-Glo reagent containing 10 mM MgCl₂ was added thereto.
   (13)The well plate was centrifuged at 1000 rpm for 10 seconds, covered, shaken for about 30 seconds and incubated at 23°C for 60 min.
   (14) 10 µL of kinase assay reagent was added.
   (15) The well plate was centrifuged at 1000 rpm for 10 seconds, and incubated at 23°C for 60 min.
   (16) Fluorescence values were measured on an Envision instrument.

### 2. Cell activity test in vitro

By CTG method, the effect of the compound to be tested on the anti-proliferation activity of cells was determined in two cell lines, HCC1954 and HDQ-P1.
Cell culture medium: complete cell culture medium (RPMI 1640 + 10% serum + 1% L-glutamine + 1% double antibody)
The specific operation steps are as follows:
   (1) HCC1954 and HDQ-P1 cells (ATCC^{®} HTB-22^{™}) were inoculated into 96-well plates, respectively, with 100 µL of cell complete medium per well (4000 cells/HDQ-P1 per well, 3500 cells/HCC1954 per well). Cells were cultured at 37°C, 5% CO₂ for 24 hours.
   (2) The cell complete medium was replaced by 100 µL of serum-free medium. The cells were starved overnight.
   (3) Compounds were prepared (the starting concentration of the compound was 10 µM, and diluted 3-fold for 8 concentrations. Each concentration of the compound was then diluted 100-fold with serum-free medium), and 25 µL of the diluted compound was added to the plate containing the cells.
   (4) The well plate was incubated at 37°C, 5% CO₂ for 72 hours (HCC1954) or 120 hours (HDQ-P1).
   (5) Subsequent operations were performed according to the instructions of the Promega CTG kit.

The results are shown in Table 2.

**Table 2 In vitro screening test results of compounds of the present disclosure**

| **Compound** | PI3K α IC₅₀ (nM) | PI3K β IC₅₀ (nM) | PI3K δ IC₅₀ (nM) | PI3K γ IC₅₀ (nM) | HCC1954 Cell IC₅₀ (nM) | HDQ-P1 Cell IC₅₀ (nM) |
|---|---|---|---|---|---|---|
| **GDC-0077** | 0.75 | 852 | 133 | 72 | 101 | NA |
| **003** | 5.52 | >10000 | >10000 | >10000 | 244 | NA |
| **006** | 18.9 | >10000 | >10000 | >10000 | 187 | NA |
| **008** | 1.22 | 705 | 16.9 | 35.9 | 207 | NA |

| | | | | | | |
|---|---|---|---|---|---|---|
| "NA" indicates that the IC₅₀ value is unable to be calculated. | | | | | | |

**Conclusion: The compound of the present disclosure can well inhibit the activity of PI3K α kinase, and has high subtype selectivity to PI3K β/ γ/ δ. In addition, in HCC1954 cells with PIK3CA mutation, the proliferation of cells can also be well inhibited.**

### Experimental Example 2: Permeability test

The permeability of the compounds of the present disclosure was determined by the membrane of MDCK cells with high expression of MDR1.

The specific operation steps are as follows:
Compounds in DMSO stock solution were diluted to 2 µM (DMSO < 1%) with a transport buffer (HBSS with 10 mM Hepes, pH 7.4) and applied to the apical side or the basolateral side of the cell monolayer. Permeation of compounds from the A to B direction or from the B to A direction was determined in duplicate. The plate was incubated for 2.5 hours in a CO₂ incubator at a temperature of 37±1°C in a saturated humidity containing 5% CO₂ without shaking. In addition, the efflux rate of each compound was also determined. Compounds were quantified by LC-MS/MS analysis based on the peak area ratio of analyte/IS.

After the transport assay, cell monolayer integrity was determined using the fluorescein exclusion test. The buffer was removed from the apical and basolateral chambers. Then 75 µL of 100 µM fluorescein transport buffer and 250 µL of transport buffer were added to the apical and basolateral chambers, respectively. The plate was incubated at 37°C, 5% CO₂ and saturated humidity for 30 min without shaking. After 30 min of incubation, 20 µL of fluorescein sample was collected from the apical side, and 60 µL of transport buffer was added. An 80 µL of fluorescein sample was then collected from the basolateral side. Relative fluorescence units (RFU) of fluorescein were measured at 425/528 nm (excitation/emission) with an Envision microplate reader. The test results are shown in Table 3.

**Table 3 The results of the permeability study of the compounds of the present disclosure**

| **Evaluation parameters** | **GDC-0077** | **008** |
|---|---|---|
| **Permeability, MDCK-MDR1, 10⁻⁶cm/s (A to B, B to A, Efflux Ratio)** | 1.99, 16.0, 8.04 | 16.9, 32.8, 1.94 |

**Conclusion: The compound of the present disclosure exhibits high permeability and low efflux properties in the MDCK-MDR1 permeability test.**

### Experimental Example 3: In Vivo Study

### 1. In vivo DMPK studies

Experimental purpose: Male CD-1 mice were used as test animals to determine plasma concentrations of compounds after a single administration and evaluate pharmacokinetic behavior.

Experimental operation: 8 healthy adult male CD-1 mice were selected, 4 for the intravenous injection group and 4 for the oral administration group. The compound to be tested was mixed with an appropriate amount of the solvent (DMSO/solvent/water (10:10:80 v/v/v)) for the intravenous injection group. The mixture solution was vortexed and sonicated to prepare a 1.0 mg/mL clear solution, which was filtered through a microporous membrane for further use. For the oral administration group, the solvent was DMSO/solvent/water (10:10:80 v/v/v). After the compound to be tested was mixed with the solvent, the mixture solution was vortexed and sonicated to prepare a 1.0 mg/mL homogeneous suspension for further use. After 1 mg/kg intravenous administration or 3 mg/kg oral administration in mice, whole blood was collected for a certain period of time to prepare plasma, and the drug concentration was analyzed by LC-MS/MS method, then the pharmacokinetic parameters were calculated using Phoenix WinNonlin software (Pharsight, USA). The results are shown in Table 4.

**Table 4 The results of the pharmacokinetic properties of the compounds of the present disclosure in mice**

| **Compound** | **Oral** | | | **Intravenous injection** | | |
|---|---|---|---|---|---|---|
| | **Cₘₐₓ (nM)** | **F%** | **DNAUC (nM.h/mpk)** | **V_{d} (L/kg)** | **Cl (mL/min/kg)** | **T_{1/2} (h)** |
| **GDC-0077** | 1765 | 50.0% | 778 | 1.09 | 26.1 | 1.20 |
| **008** | 9345 | 89.1% | 6441 | 0.372 | 5.30 | 1.13 |

**Conclusion: The compound of the present disclosure exhibits high exposure, low clearance rate, and good oral bioavailability in mice.**

### 2. In vivo plasma/brain tissue distribution study

Experimental purpose: Male SD rats were used as test animals to determine plasma concentrations of compounds and drug concentrations of brain tissue and cerebrospinal fluid after a single administration, and to evaluate the brain entry of the compound of the present disclosure.

Experimental operation: 12 healthy adult male SD rats were selected. The compound to be tested was mixed with an appropriate amount of solvent (DMSO/solvent/water (10:10:80 v/v/v)) for the oral administration group. The mixture was vortexed and sonicated to prepare a 1.0 mg/mL clear solution for further use. After oral administration of 10 mg/kg to rats, whole blood, brain tissue, and cerebrospinal fluid for a certain period of time were collected to prepare plasma, brain homogenate, and cerebrospinal fluid. The drug concentration was analyzed by LC-MS/MS method, and the pharmacokinetic parameters were calculated by Phoenix WinNonlin software (Pharsight, USA). The results are shown in Table 5.

**Table 5 The results of the study on the distribution of compounds of the present disclosure in rat plasma/brain tissue**

| **Oral administration 10 mpk in rats** | | **GDC-0077** | **008** |
|---|---|---|---|
| **Cmax(nM), Tmax(h), T_{1/2}(h), AUC(nM * h)** | **Brain tissue** | 147, 1.00, ND, 342 | 1043, 0.25, 1.45, 2082 |
| | **Cerebrospinal fluid** | 60.4, 1.00, ND, 128 | 552, 0.25, 1.38, 1077 |
| | **Plasma** | 1525, 0.25, 1.32, 3117 | 10295, 0.25, 1.72, 21717 |
| **Rat plasma-protein binding, unbound%** | | 51.5% | 23.9% |
| **Cerebrospinal fluid/plasma free drug ratio** | | 0.080 | 0.21 |

| | | | |
|---|---|---|---|
| Note: ND means that it is unable to be calculated. | | | |

**Conclusion: The compound of the present disclosure exhibits higher brain drug exposure levels in rats.**

## Claims

1. A compound of formula (II) or a pharmaceutically acceptable salt thereof, 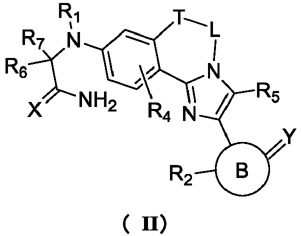 wherein
T is selected from O and S;
L is selected from -C₁₋₃ alkyl- and -C₁₋₃ alkyl-cyclopropyl-;
R₁ is selected from H and C₁₋₃ alkyl, and the C₁₋₃ alkyl is optionally substituted by 1, 2, or 3 Rₐ;
R₂ is selected from H, F, Cl, Br, I, OH, and C₁₋₃ alkyl, and the C₁₋₃ alkyl is optionally substituted by 1, 2, or 3 R_{b};
X and Y are each independently selected from O and NR₃, and X and Y are not selected from O at the same time;
R₃ is independently selected from H, OH, CN, C₁₋₃ alkyl, C₁₋₃ alkoxy, and -O-C₃₋₅ cycloalkyl, and the C₁₋₃ alkyl, C₁₋₃ alkoxy, and -O-C₃₋₅ cycloalkyl are optionally substituted by 1, 2, or 3 R_{c};
R₄ and R₅ are selected from H, F, Cl, Br, I, OH, and C₁₋₃ alkyl;
R₆ is selected from H and C₁₋₃ alkyl;
R₇ is selected from C₁₋₃ alkyl, C₁₋₃ alkoxy, and 3- to 5-membered heterocycloalkyl;
or, R₆, R₇ together with the carbon atom they share form a 3- to 5-membered heterocycloalkyl;
or, R₁, R₇ together with the atom to which they are attached form a 3- to 5-membered heterocycloalkyl;
ring B is selected from 4- to 8-membered heterocycloalkyl, and the 4- to 8-membered heterocycloalkyl is optionally substituted by 1, 2, or 3 R_{d};
Rₐ, R_{b}, R_{c}, and R_{d} are each independently selected from F, Cl, Br, and I.

2. The compound or the pharmaceutically acceptable salt thereof according to claim 1, wherein R₁ is selected from H and CH₃, and the CH₃ is optionally substituted by 1, 2, or 3 Rₐ.

3. The compound or the pharmaceutically acceptable salt thereof according to claim 2, wherein R₁ is selected from H, CH₃, CH₂F, CHF₂, and CF₃.

4. The compound or the pharmaceutically acceptable salt thereof according to claim 1, wherein R₂ is selected from H, F, Cl, Br, I, OH, and CH₃, and the CH₃ is optionally substituted by 1, 2, or 3 R_{b}.

5. The compound or the pharmaceutically acceptable salt thereof according to claim 4, wherein R₂ is selected from H, F, Cl, Br, I, OH, CH₃, CH₂F, CHF₂, and CF₃.

6. The compound or the pharmaceutically acceptable salt thereof according to claim 1, wherein R₃ is independently selected from H, OH, CN, CH₃, CH₂CH₃, OCH₃, OCH₂CH₃, - OCH(CH₃)₂, -O-cyclopropyl, and -O-cyclobutyl, and the CH₃, CH₂CH₃, OCH₃, OCH₂CH₃, - OCH(CH₃)₂, -O-cyclopropyl, and -O-cyclobutyl are optionally substituted by 1, 2, or 3 R_{c}.

7. The compound or the pharmaceutically acceptable salt thereof according to claim 6, wherein R₃ is independently selected from H, OH, CN, CH₃, CH₂CH₃, OCH₃, OCH₂CH₃, - OCH(CH₃)₂, -O-cyclopropyl, and -O-cyclobutyl.

8. The compound or the pharmaceutically acceptable salt thereof according to claim 1 or 7, wherein X and Y are each independently selected from O, NH, NOH, NCN, N-CH₃, N-OCH₃, N-OCH₂CH₃, N- OCH(CH₃)₂, N-O-cyclopropyl, and N-O-cyclobutyl.

9. The compound or the pharmaceutically acceptable salt thereof according to claim 1, wherein R₄ and R₅ are selected from H, F, Cl, Br, I, OH, and CH₃.

10. The compound or the pharmaceutically acceptable salt thereof according to claim 1, wherein R₇ is selected from CH₃, CH(CH₃)₂, OCH₃, and oxetanyl.

11. The compound or the pharmaceutically acceptable salt thereof according to claim 1, wherein R₆, R₇ together with the carbon atom they share form an oxetanyl.

12. The compound or the pharmaceutically acceptable salt thereof according to claim 1, wherein R₁, R₇ together with the atom to which they are attached form an azetidinyl and a pyrrolidinyl.

13. The compound or the pharmaceutically acceptable salt thereof according to claim 1, wherein L is selected from -CH₂CH₂-, -CH(CH₃)CH₂-, and

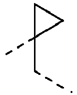

14. The compound or the pharmaceutically acceptable salt thereof according to claim 1, wherein ring B is selected from 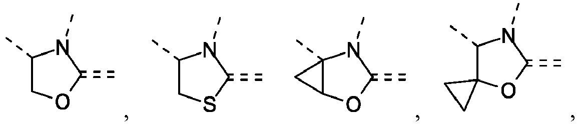 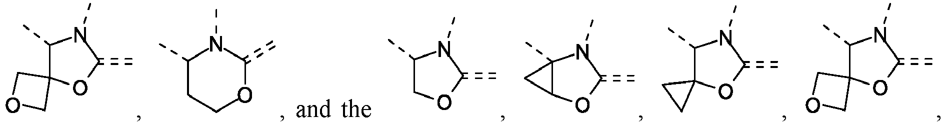 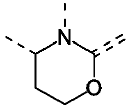 are optionally substituted by 1, 2, or 3 R_{d}.

15. The compound or the pharmaceutically acceptable salt thereof according to claim 14, wherein ring B is selected from

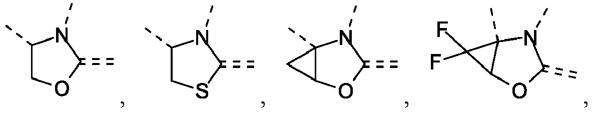

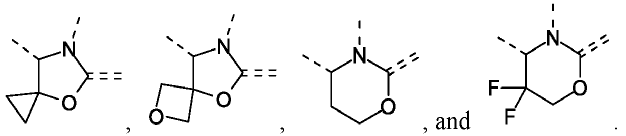

16. The compound or the pharmaceutically acceptable salt thereof according to any one of claims 1 to 7, selected from 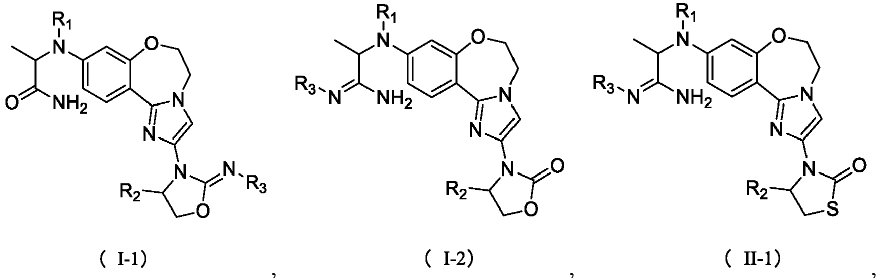 wherein
R₁ is as defined in any one of claims 1 to 3;
R₂ is as defined in any one of claims 1, 4, or 5;
R₃ is as defined in any one of claims 1, 6, or 7.

17. The compound or the pharmaceutically acceptable salt thereof according to claim 16, selected from 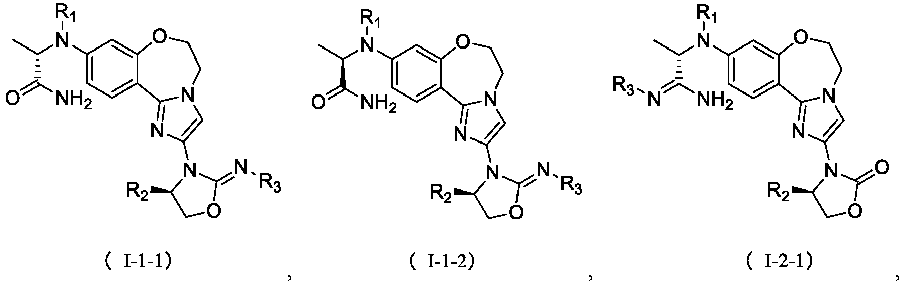 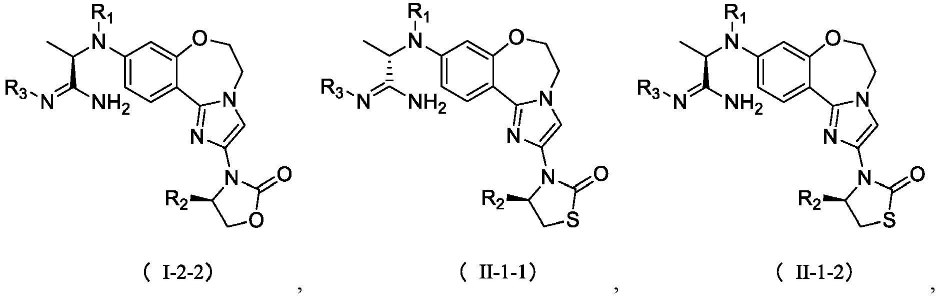 wherein
R₁, R₂, and R₃ are as defined in claim 16.

18. A compound of the following formula or a pharmaceutically acceptable salt thereof,

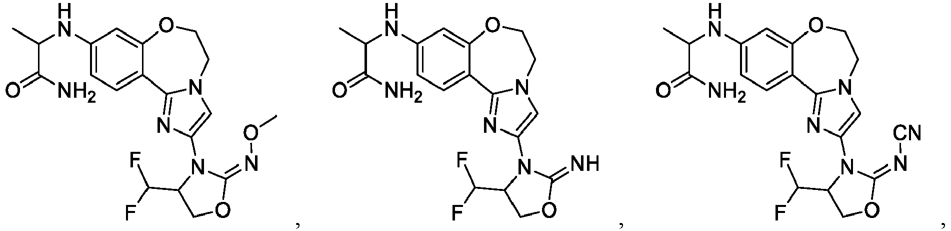

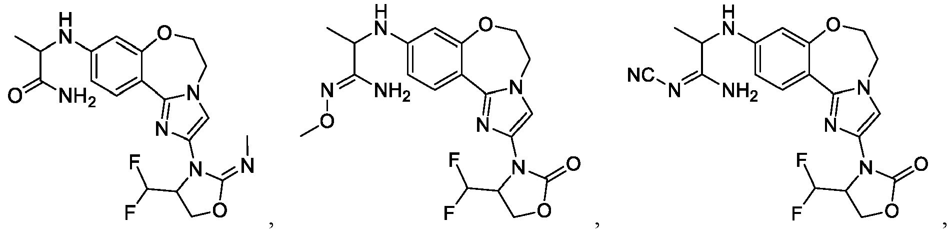

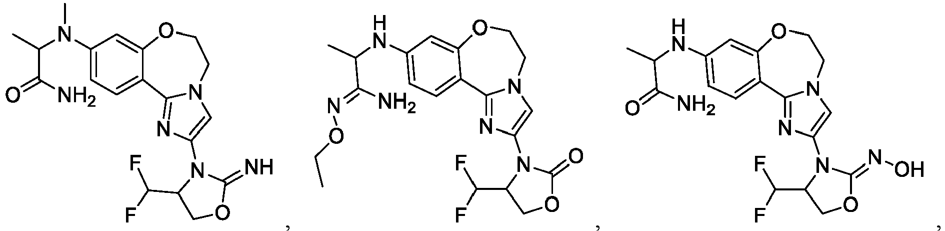

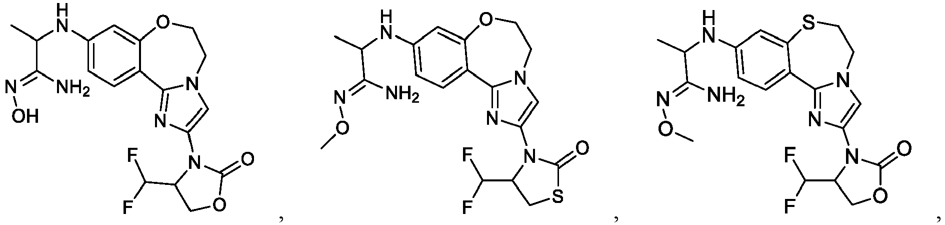

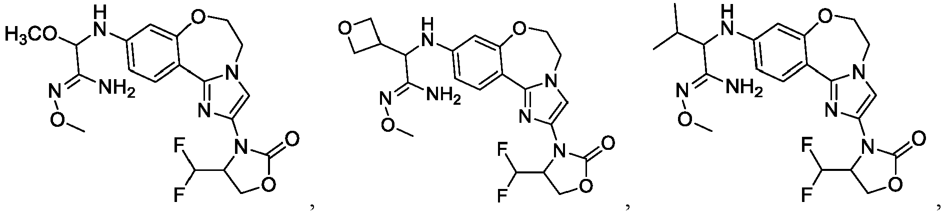

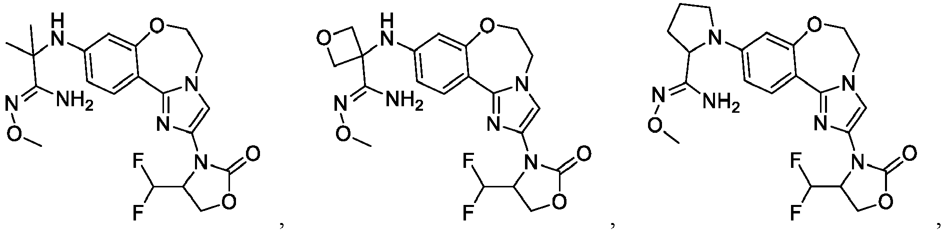

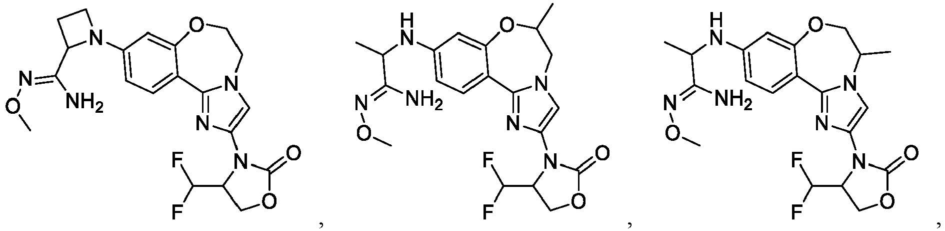

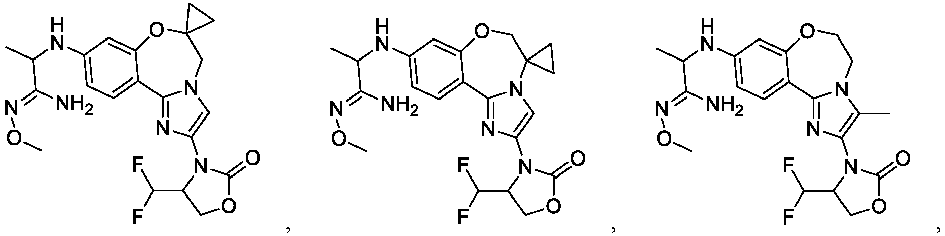

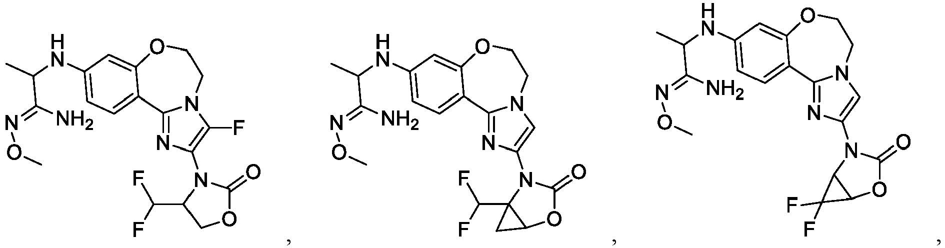

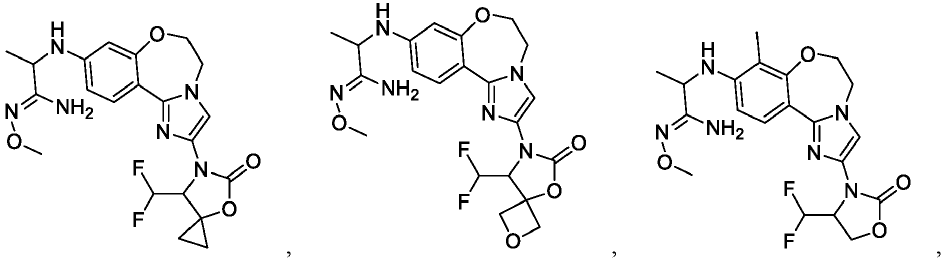

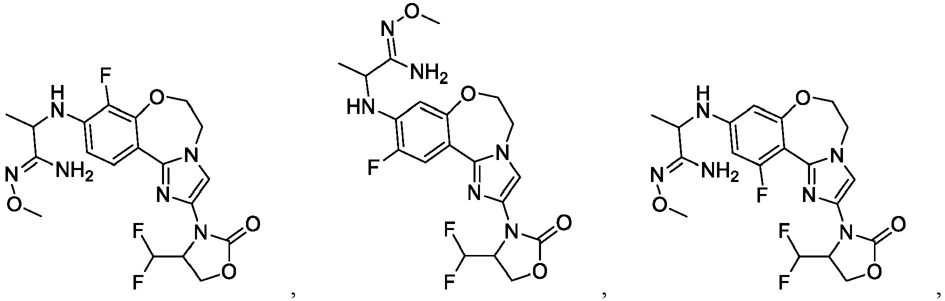

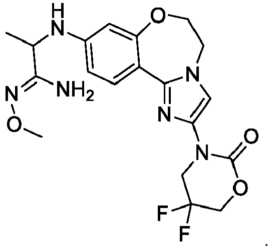

19. The compound or the pharmaceutically acceptable salt thereof according to claim 18, selected from
